(19) Europäisches Patentamt
European Patent Office
Office européen des brevets

(11) EP 2 716 630 A1

(12) EUROPEAN PATENT APPLICATION
published in accordance with Art. 153(4) EPC

(43) Date of publication:
**09.04.2014 Bulletin 2014/15**

(21) Application number: **12792034.6**

(22) Date of filing: **17.05.2012**

(51) Int Cl.:
***C07D 209/52*** (2006.01) ***C07C 205/29*** (2006.01)
***C07C 217/44*** (2006.01) ***C07C 271/16*** (2006.01)
***C07C 271/18*** (2006.01) ***C07D 317/16*** (2006.01)
***C07D 317/28*** (2006.01)

(86) International application number:
**PCT/JP2012/063243**

(87) International publication number:
**WO 2012/165268 (06.12.2012 Gazette 2012/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2011 JP 2011122149**
**12.01.2012 JP 2012004149**

(71) Applicant: **Sumitomo Chemical Company Limited**
**Tokyo 104-8260 (JP)**

(72) Inventors:
- **IKEMOTO, Tetsuya**
**Osaka-shi**
**Osaka 555-0021 (JP)**
- **LUTETE, Leopold Mpaka**
**Osaka-shi**
**Osaka 555-0021 (JP)**
- **AIKAWA, Toshiaki**
**Osaka-shi**
**Osaka 555-0021 (JP)**

(74) Representative: **Nieuwenhuys, William Francis**
**Marks & Clerk LLP**
**90 Long Acre**
**London**
**WC2E 9RA (GB)**

# (54) PROCESS FOR PRODUCING ESTER COMPOUND

(57) Compound (1) or a salt that is useful as an intermediate for the production of a medicine, an agrochemical or the like can be produced by a process comprising steps A to G as mentioned below:

[Step A] A step of reacting an aldehyde compound (2) with nitromethane to produce a nitroaldehyde compound (3);
[Step B] A step of reacting the nitroaldehyde compound (3) with an alcohol to produce a nitroacetal compound (4);
[Step C] A step of reducing the nitroacetal compound (4) to produce an aminoacetal compound (5);
[Step D] A step of protecting an amino group in the aminoacetal compound (5) to produce a protected aminoacetal compound (6);
[Step E] A step of treating the protected aminoacetal compound (6) with an acid and aubsequently with a base and then reacting the resultant product with a cyanating agent to produce a nitrile compound (7);
[Step F] A step of hydrolyzing the nitrile compound (7) to produce a protected amino acid compound (8); and
[Step G] A steep of substituting a group $R^5$ in the protected amino acid compound (8) by a hydrogen atom and protecting a carboxyl group therein.

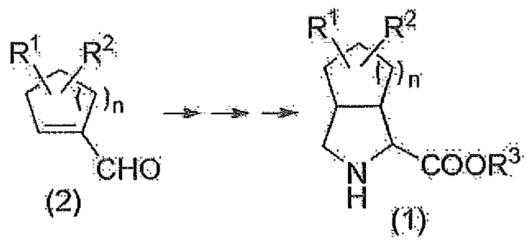
(2) (1)

EP 2 716 630 A1

## Description

### TECHNICAL FIELD

The present invention relates to a process for producing an ester compound useful as an intermediate to produce medicines, agrochemicals, and the like.

### BACKGROUND ART

An ester compound represented by Formula (1)

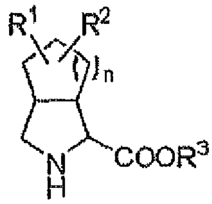

(1)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group; $R^3$ represents a protective group of a carboxyl group; and n represents 1 or 2; is known as an intermediate to produce medicines, agrochemicals, and the like, for example, an intermediate to produce a hepatitis C therapeutic agent, Telaprevir.

As a process for producing the compound represented by Formula (1), for example, WO 2007/022459 and WO 2010/126881 disclose a process to obtain a compound represented by Formula (1a) from 3-azabicyclo[3.3.0]nonane hydrochloride.

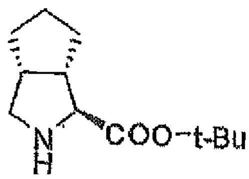

(1a)

The present invention provides a novel process for producing an ester compound represented by Formula (1), and the like.

### SUMMARY OF THE INVENTION

The present invention includes the following.

[1] A process for producing an ester compound represented by the above Formula (1) (ester compound (1)) or a salt thereof, the process comprising Steps A to G defined below:

[Step A] a step of reacting an aldehyde compound represented by Formula (2) (aldehyde compound (2))

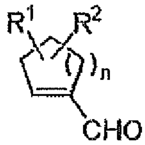

(2)

wherein $R^1$, $R^2$, and n are each as defined above,
with nitromethane in the presence of a pyrrolidine compound to produce a nitroaldehyde compound represented by Formula (3) given below;

[Step B] a step of reacting the nitroaldehyde compound represented by Formula (3) (nitroaldehyde compound (3))

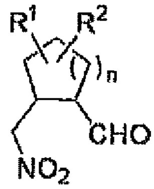

(3)

wherein $R^1$, $R^2$, and n are each as defined above,
with an alcohol to produce a nitroacetal compound represented by Formula (4) given below;
[Step C] a step of reducing the nitroacetal compound represented by Formula (4) (nitroacetal compound (4))

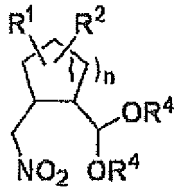

(4)

wherein $R^1$, $R^2$, and n are each as defined above, and $R^4$s each represent an alkyl group or the two $R^4$s are combined together to represent an alkylene group,
to produce an aminoacetal compound represented by Formula (5) given below or a salt thereof;
[Step D] a step of protecting an amino group in the aminoacetal compound represented by Formula (5) (aminoacetal compound (5))

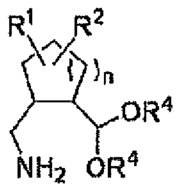

(5)

wherein $R^1$, $R^2$, $R^4$, and n are each as defined above,
or a salt thereof to produce a protected aminoacetal compound represented by Formula (6) given below or a salt thereof; [Step E] a step of treating the protected aminoacetal compound represented by Formula (6) (protected aminoacetal compound (6))

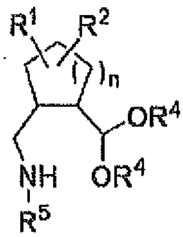

(6)

wherein $R^1$, $R^2$, $R^4$, and n are each as defined above, and $R^5$ represents a protecting group of an amino group, or a salt thereof with an acid and subsequently with a base and then reacting the resultant product with a cyanating agent to produce a nitrile compound represented by Formula (7) given below or a salt thereof;
[Step F] a step of hydrolyzing the nitrile compound represented by Formula (7)

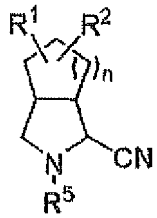

(7)

wherein $R^1$, $R^2$, $R^5$, and n are each as defined above,

or a salt thereof to produce a protected amino acid compound represented by Formula (8) given below or a salt thereof; and [Step G] a step of substituting a group represented by $R^5$ contained in the protected amino acid compound represented by Formula (8)

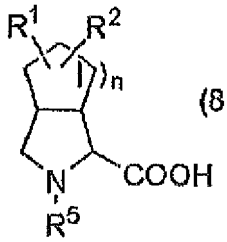

(8)

wherein $R^1$, $R^2$, $R^5$, and n are each as defined above,

or a salt thereof by a hydrogen atom and protecting a carboxyl group therein.

[2] The production process according to [1], wherein Step A is a step of reacting the aldehyde compound represented by Formula (2) with nitromethane in the presence of water.

[3] The production process according to [1] or [2], wherein Step A is a step of reacting the aldehyde compound represented by Formula (2) with nitromethane further in the presence of a carboxylic acid.

[4] The production process according to any of [1] to [3], wherein the pyrrolidine compound in Step A is a compound represented by Formula (9) (catalyst (9))

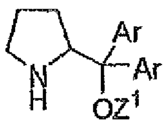

(9)

wherein Ar represents an optionally substituted aryl group, and $Z^1$ represents an alkyl group or a trialkylsilyl group (each of the alkyl groups in the trialkylsilyl group may be the same or different).

[5] The production process according to [4], wherein Ar is phenyl and $Z^1$ is a trimethylsilyl group.

[6] The production process according to any of [1] to [5], wherein the aldehyde compound represented by Formula (2) is 1-formylcyclopentene.

[7] A process for producing a protected aminoalcohol compound represented by Formula (6bX)

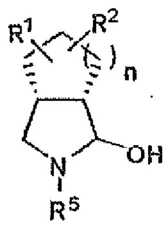

(6bX)

wherein $R^1$, $R^2$, $R^5$, and n are each as defined below, the process comprising a step of treating a protected aminoacetal compound represented by Formula (6X)

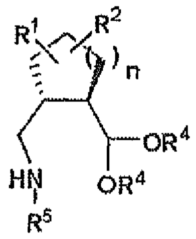

(6X)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, $R^5$ represents a protecting group of an amino group, and n represents 1 or 2, or a salt thereof with an acid to form a protected aminoaldehyde compound represented by Formula (6aX)

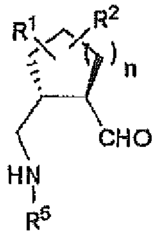

(6aX)

wherein $R^1$, $R^2$, $R^5$, and n are each as defined above, and a step of treating the protected aminoaldehyde compound represented by Formula (6aX) with a base.

[8] The production process according to [7], wherein the base is 1,8-diazabicyclo[5.4.0]undec-7-ene or a metal alkoxide.

[9] A nitroacetal compound represented by Formula (4)

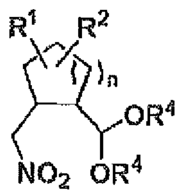

(4)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, and n represents 1 or 2.

[10] An aminoacetal compound represented by Formula (5)

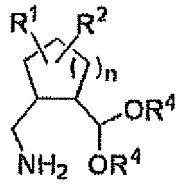

(5)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group

group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, and n represents 1 or 2, or a salt thereof.

[11] A protected aminoacetal compound represented by Formula (6)

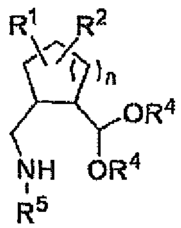

(6)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,

or a salt thereof.

[12] A nitrile compound represented by Formula (7)

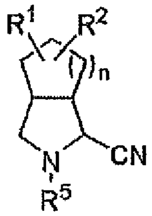

(7)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,

or a salt thereof.

[13] A protected amino acid compound represented by Formula (8)

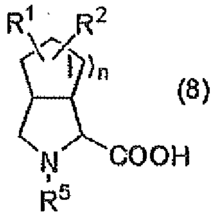

(8)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,
or a salt thereof.
[14] A protected aminoaldehyde compound represented by Formula (6a)

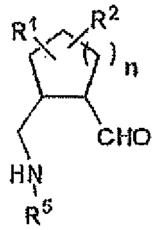

(6a)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,
or a salt thereof.
[15] A protected aminoalcohol compound represented by Formula (6b)

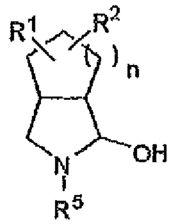

(6b)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,
or a salt thereof.

MODE FOR CARRYING OUT THE INVENTION

In the present invention, examples of an "alkyl group" include straight chain or branched $C_1$ to $C_{10}$ alkyl groups and specifically include methyl, ethyl, propyl, isopropyl, butyl, sec-butyl, tert-butyl, pentyl, hexyl, heptyl, octyl, nonyl, and decyl. Preferred are methyl, ethyl, isopropyl, sec-butyl, tert-butyl, and the like, and particularly preferred are methyl, ethyl, and the like.
Examples of an "alkoxy group" include straight chain or branched $C_1$ to $C_{10}$ alkoxy groups and specifically include methoxy, ethoxy, propoxy, isopropoxy, butoxy, sec-butoxy, tert-butoxy, pentoxy, hexyloxy, heptyloxy, octyloxy, nonyloxy, and decyloxy. Preferred are methoxy, ethoxy, isopropoxy, sec-butoxy, tert-butoxy, and the like, and particularly preferred are methoxy, ethoxy, and the like.
Examples of an "alkenyl group" include straight chain or branched $C_3$ to $C_{10}$ alkenyl groups and specifically include allyl, butenyl, pentenyl, hexenyl, heptenyl, octenyl, nonenyl, and decenyl. Preferred are allyl, 2-butenyl, and the like. Examples of an "alkenyloxy group" include straight chain or branched $C_3$ to $C_{10}$ alkenyloxy groups and specifically include allyloxy, butenyloxy, pentenyloxy, hexenyloxy, heptenyloxy, octenyloxy, nonenyloxy, and decenyloxy. Preferred are allyloxy, 2-butenyloxy, and the like.
Examples of a "cycloalkyl group" include $C_4$ to $C_{10}$ cycloalkyl groups and specifically include cyclobutyl, cyclopentyl, cyclohexyl, cycloheptyl, cyclooctyl, cyclononyl, and cyclodecyl.

Examples of a "cycloalkoxy group" include $C_4$ to $C_{10}$ cycloalkoxy groups and specifically include cyclobutyloxy, cyclopentyloxy, cyclohexyloxy, cycloheptyloxy, cyclooctyloxy, cyclononyloxy, and cyclodecyloxy.

Examples of an "aryl group" include $C_6$ to $C_{10}$ aryl groups and specifically include phenyl and naphthyl. Preferred is phenyl.

Examples of a "heteroaryl group" include 5- or 6-membered heteroaryl groups containing 1, 2 or 3 hetero atoms independently selected from a nitrogen atom, an oxygen atom, and a sulfur atom. Specific examples include thiazolyl, isothiazolyl, thiadiazolyl, pyridyl, pyrazinyl, pyridazinyl, pyrazolyl, imidazolyl, pyrimidinyl, oxazolyl, isoxazolyl, oxadiazolyl, and triazolyl.

Examples of "halogen" include fluorine, chlorine, bromine, and iodine, and preferred are fluorine and chlorine.

Examples of the "protective group of an amino group" of $R^5$ include protective groups of an amino group known in the field of organic chemistry, for example, those disclosed in "Protective Groups in Organic Synthesis", by T. W. Greene and P. G. M. Wuts (published by Wiley-Interscience, 4th edition, 2006). Preferred as the protective group are protective groups capable of being deprotected under conditions different from the conditions for the protective group of a carboxyl group of $R^3$, and more preferred are urethane type protective groups and amide type protective groups. Particularly preferred are tert-butoxycarbonyl, benzyloxycarbonyl, aryloxycarbonyl, acetyl, benzyl, and the like. The substituent in the "optionally substituted amino group" of $R^1$ and $R^2$ is preferably a protective group, and specific examples thereof are the same as those provided here.

Examples of the "protective group of a carboxyl group" of $R^3$ include protective groups of a carboxyl group known in the field of organic chemistry, for example, those disclosed in "Protective Groups in Organic Synthesis", by T. W. Greene and P. G. M. Wuts (published by Wiley-Interscience, 4th edition, 2006). Preferred as the protective group are ester type protective groups capable of being deprotected under conditions different from the conditions for the protective group of an amino group of $R^5$, and more preferred are an optionally substituted alkyl group, an optionally substituted alkenyl group, an optionally substituted cycloalkyl group, and the like, even more preferred are an optionally substituted straight chain or branched $C_1$ to $C_{10}$ alkyl group, an optionally substituted straight chain or branched $C_3$ to $C_{10}$ alkenyl group, and an optionally substituted $C_4$ to $C_{10}$ cycloalkyl group, and particularly preferred are methyl, ethyl, and the like.

Examples of the "substituent" with which an alkyl group, an alkoxy group, an alkenyl group, an alkenyloxy group, a cycloalkyl group, a cycloalkoxy group, an aryl group, and a heteroaryl group are substituted include an alkyl group, hydroxy, halogen, nitro, a halogen-substituted alkyl group, an alkoxy group, a cycloalkoxy group, an aryl group, and a heteroaryl group. Examples of the "substituent" of an amino group include an alkyl group, a halogen-substituted alkyl group, an aryl group, and a cycloalkoxy group as well as the above-mentioned preferred protective groups. In the case of having a plurality of substituents, each of the substituents may be the same or different.

According to the present invention, it is possible to produce an ester compound (1) or a salt thereof by a method comprising Step A through Step G.

[Step A]

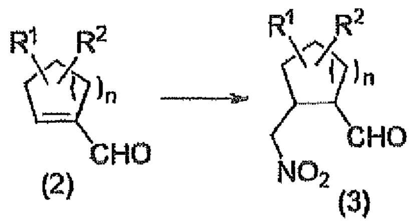

(2) (3)

wherein $R^1$, $R^2$, and n are each as defined above.

In Step A, a nitroaldehyde compound (3) is produced by reacting an aldehyde compound (2) with nitromethane in the presence of a pyrrolidine compound.

The aldehyde compound (2) is commercially available or alternatively can be prepared easily from a commercially available compound.

The amount of nitromethane used is usually 1 to 20 mol, preferably 1 to 10 mol, and more preferably 1 to 3 mol per 1 mol of the aldehyde compound (2).

The pyrrolidine compound is a cyclic amine compound having a pyrrolidine ring and is a compound capable of acting as a catalyst in the reaction of the aldehyde compound (2) and nitromethane. Preferred is a compound represented by Formula (9)

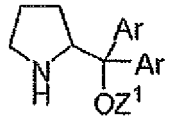 (9)

wherein Ar represents an optionally substituted aryl group; and $Z^1$ represents an alkyl group or a trialkylsilyl group (each of the alkyl groups in this trialkylsilyl group may be the same or different). The aryl group of Ar is preferably a phenyl group, and examples of the substituent of the aryl group include an alkyl group, halogen, a halogen-substituted alkyl group, and nitro, and preferably include methyl, ethyl, fluorine, chlorine, and trifluoromethyl. A preferred Ar may be unsubstituted phenyl. $Z^1$ is preferably methyl, ethyl, or trimethylsilyl, and more preferably trimethylsilyl. The catalyst (9) has an asymmetric carbon atom and can be present as an optically active isomer or a recemic mixture due to the asymmetric carbon atom. The use of an optically active catalyst (9) can cause asymmetric induction at the β-position of the nitroaldehyde compound (3) in an extremely high selectivity. The catalyst (9) is usually used in a catalytic amount though it may be used in a large amount. For example, the amount thereof used is 0.1% mol to 50% mol, preferably 0.5% mol to 30% mol, and more preferably 1% mol to 5% mol per 1 mol of the aldehyde compound (2).

Examples of the reaction solvent include an alcohol, an ether, a sulfoxide, an amide, a halogenated hydrocarbon, water, or a mixture thereof. Examples of the alcohol include methanol, ethanol, propanol, 2-propanol, sec-butyl alcohol, tert-butyl alcohol, pentanol, neopentyl alcohol, sec-pentyl alcohol, hexanol, and ethylene glycol. Examples of the ether include tetrahydrofuran (THF), dioxane, dimethoxyethane, diethylene glycol dimethyl ether, and polyethylene glycol. Examples of the sulfoxide include dimethyl sulfoxide. Examples of the amide include N,N-dimethylformamide and N,N-dimethylacetamide. Examples of the halogenated hydrocarbon include methylene chloride, chloroform, and dichloroethane. Alcohols are preferred for the reaction solvent, $C_1$ to $C_6$ alcohols are more preferred, methanol and 2-propanol are even more preferred, and methanol is particularly preferred. The amount of the reaction solvent is, for example, preferably 0.5 mL to 50 mL, more preferably 1 mL to 10 mL, and even more preferably 2 mL to 5 mL per 1 g of the aldehyde compound (2).

In Step A, adding water is also preferred. The addition of water can increase the reaction rate. In Step A, reducing the amount of the pyrrolidine compound used tends to decrease the reaction rate, and the addition of water can prevent the reaction rate from decreasing. Further, adding water also serves to reduce the amount of the pyrrolidine compound used. The amount of the water to be added is, for example, preferably 0.01 mol to 20 mol, more preferably 0.1 mol to 5 mol, and even more preferably 1 mol to 3 mol per 1 mol of the aldehyde compound (2).

In the reaction of Step A, adding a carboxylic acid is also preferred. Examples of the carboxylic acid include alkanoic acids such as acetic acid and propionic acid, and optionally substituted benzoic acid. Preferred are acetic acid, benzoic acid, and the like, and more preferred is acetic acid. The amount of the carboxylic acid used is, for example, 0.05 mol to 1 mol, preferably 0.1 mol to 0.5 mol, and more preferably 0.1 mol to 0.3 mol pre 1 mol of the pyrrolidine compound. In the case where the carboxylic acid is used in a large amount, there may be by-produced a dialkylacetal resulting from a reaction of the aldehyde compound (2) with an alcohol, which is a reaction solvent. It is also preferred to adjust the amount of the carboxylic acid used while checking the generation of the by-product.

The reaction temperature is, for example, -40°C to 50°C, preferably -20°C to 40°C, and more preferably 0°C to 30°C. A lower temperature is preferred because it can suppress side reactions. The reaction time may be chosen appropriately by checking the process of the reaction and the amount of by-products, and it can be chosen within the range of from 30 minutes to 50 hours, preferably from 1 hour to 25 hours. After the completion of the reaction, nitroaldehyde can be isolated by conducting post treatment in accordance with an ordinary method.

While there are no particular limitations with respect to the method or the order of charging materials into a reaction vessel, preferred is a method involving adding a pyrrolidine compound, nitromethane, and, as required, water and a carboxylic acid to a solvent and then dropping a aldehyde compound (2) into the resulting mixture.

The aldehyde compound (2) to be used for Step A can be produced, for example, by bringing an α,ω-alkanediol (e.g., 1,6-hexanediol) according to the desired n into contact with an oxidizing agent in the presence of a bromide and an N-oxyl compound in a solvent. Aprotic solvents can be used as the above-mentioned solvent; particularly, halogenated hydrocarbons are preferred, and dichloromethane and chloroform are more preferred. The amount of the solvent used is usually 5 to 50 L, preferably 10 to 30 L per 1 kg of the α,ω-alkanediol.

Metal bromides, such as sodium bromide, quaternary ammonium bromides, such as tetrabutylammonium bromide, and the like can be used for the above-mentioned bromide. Especially, potassium bromide is preferred. The amount of the bromide used is usually 0.05 to 1 mol, preferably 0.1 to 0.5 mol per 1 mol of the α,ω-alkanediol.

2,2,6,6-Tetramethylpiperidine N-oxyl (TEMPO) is preferred as the N-oxyl compound. The amount of the N-oxyl compound used is usually 0.003 to 0.1 mol, preferably 0.005 to 0.03 mol per 1 mol of the α,ω-alkanediol.

Sodium hypochlorite is preferred for the oxidizing agent, and it is preferred to use sodium hypochlorite in the form of an aqueous solution. The amount of the oxidizing agent used is usually 2 to 4 mol, preferably 2 to 3 mol per 1 mol of the α,ω-alkanediol.

Preferably, a pH regulating agent is added to the reaction system of the α,ω-alkanediol and the oxidizing agent. Examples of the pH regulating agent include disodium hydrogen phosphate, sodium phosphate, dipotassium hydrogen phosphate, potassium phosphate, and potassium hydrogen carbonate, and especially, sodium hydrogen carbonate is preferred. The amount of the pH regulating agent used is usually 0.5 to 3 mol, preferably 1 to 2 mol per 1 mol of the α,ω-alkanediol.

The reaction temperature of the reaction of the α,ω-alkane diol with the oxidizing agent is usually within the range of from -20 to 40°C, preferably within the range of from 0 to 20°C. The reaction time is usually within the range of from 0.1 to 5 hours, preferably within the range of from 0.5 to 2 hours.

[Step B]

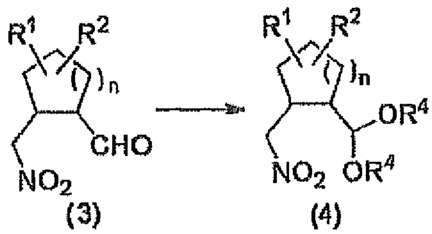

(3) (4)

wherein $R^1$, $R^2$, and n are each as defined above; and $R^4$s each represent an alkyl group or the two $R^4$s are combined together to represent an alkylene group.

In Step B, a nitroacetal compound (4) is produced by reacting a nitroaldehyde compound (3) with an alcohol. The nitroaldehyde compound (3) is acetalized with the alcohol, and the acetalization can be conducted in accordance with an ordinary method, for example, by reacting the nitroaldehyde compound (3) with the alcohol under an acidic condition.

Any alcohol that is usually used for the protection of an aldehyde can be used, and examples thereof include methanol, ethanol, ethylene glycol, propylene glycol, and 2,2-dimethylpropylene glycol, and ethylene glycol is preferred. The amount of the alcohol used is usually 1 mol to 30 mol, preferably 1.1 mol to 10 mol, and more preferably 1.2 mol to 5 mol per 1 mol of the nitroaldehyde compound (3).

In the case of conducting the acetalization under an acidic condition, examples of the acid to be used include sulfonic acids (methanesulfonic acid, p-toluenesulfonic acid, benzenesulfonic acid, and the like) and inorganic acids (sulfuric acid, phosphoric acid, hydrochloric acid, hydrobromic acid, and the like), and preferred are sulfonic acids. The amount of the acid used is usually 0.01% mol to 10% mol, preferably 0.1% mol to 5% mol, and more preferably 1% mol to 3% mol per 1 mol of the nitroaldehyde compound (3).

It is preferred to remove water from the reaction system in order to complete the reaction, and for this purpose, it is preferred to remove water with a Dean-Stark trap or the like using a solvent that boils azeotropically with water and is immiscible with water (e.g., toluene and benzene) or to add a dehydrating agent. Particularly preferred as the dehydrating agent is a trialkoxymethane. In the case of using a dehydrating agent, the amount thereof used is usually 0.5 to 5 mol, preferably 1 to 3 mol per 1 mol of the nitroaldehyde compound (3).

The reaction temperature is, for example, 0°C to 130°C, preferably 20°C to 120°C. The reaction time may be chosen appropriately by checking the process of the reaction and the amount of by-products, and it is 30 minutes to 40 hours, preferably 1 hour to 20 hours. After the completion of the reaction, post treatment is conducted in accordance with an ordinary method.

[Step C]

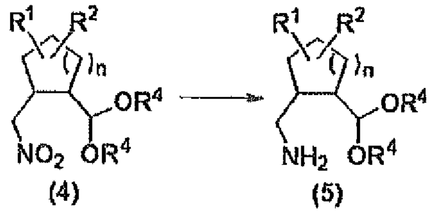

(4) (5)

wherein $R^1$, $R^2$, $R^4$, and n are each as defined above.

In Step C, an aminoacetal compound (5) or a salt thereof is produced by reducing the nitroacetal compound (4).

For the reduction, there is used a reducing agent that can be used for the reduction of a nitro group and does not make an acetal leave. For example, reduction can be conducted by the addition of hydrogen. In this case, the reduction can be conducted by stirring in the presence of a catalyst (Pd/carbon, palladium hydroxide, platinum, or the like) under a hydrogen atmosphere at 0°C to 50°C.

The aminoacetal compound (5) can also be converted into a salt thereof in accordance with an ordinary method. Examples of the salt include inorganic acid salts (a hydrochloride, a sulfate, a phosphate, a nitrate, and the like) and organic acid salts (an acetate, a citrate, a p-toluenesulfonate, a methanesulfonate, and the like).

[Step D]

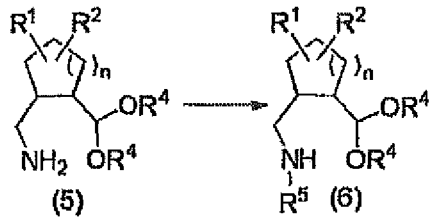

wherein $R^1$, $R^2$, $R^4$, $R^5$, and n are each as defined above.

In Step D, a protected acetal compound (6) or a salt thereof is produced by protecting an amino group of the aminoacetal compound (5) or a salt thereof.

While examples of the "protective group of a amino group" include those specifically disclosed in "Protective Groups in Organic Synthesis", byT. W. Greene and P. G. M. Wuts (published by Wiley-Interscience, 4th edition, 2006), the protective group can be introduced in accordance with an ordinary method such as a method disclosed in the above-cited book. When using a urethane type protective group, an amide type protective group, or the like, the introduction of the protective group can be conducted, for example, by a method involving reacting the group with its corresponding chloride in the presence of a base, or a method involving reacting the group with a proper aldehyde compound to form a Schiff base and then reducing (hydrogenating) the Schiff base.

The protected acetal compound (6) can also be converted into a salt thereof in accordance with an ordinary method. Examples of the salt include inorganic acid salts (a hydrochloride, a sulfate, a phosphate, a nitrate, and the like) and organic acid salts (an acetate, a citrate, a p-toluenesulfonate, a methanesulfonate, and the like).

[Step E]

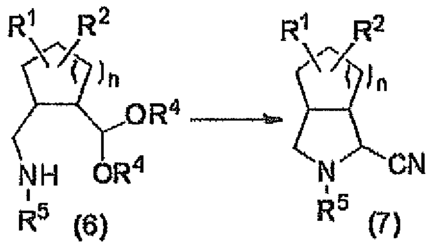

wherein $R^1$, $R^2$, $R^5$, and n are each as defined above.

In step E, a nitrile compound (7) or a salt thereof is produced by treating the protected acetal compound (6) or a salt thereof with an acid and subsequently with a base, and then further reacting a cyanating agent. Particularly preferred is that $R^5$ of the protected acetal compound (6) is an optionally substituted alkyloxycarbonyl group, an optionally substituted aryloxycarbonyl group, or an optionally substituted aralkyl group.

In Step E, the protected aminoacetal compound represented by Formula (6) or a salt thereof is treated with an acid first, thereby being converted into a protected aminoaldehyde compound represented by Formula (6a)

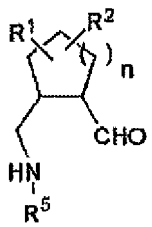

(6a)

wherein $R^1$, $R^2$, $R^5$, and n are each as defined above. The protected aminoaldehyde compound represented by Formula (6a) is converted by base treatment into a protected aminoalcohol compound represented by Formula (6b)

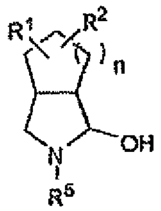

(6b)

wherein $R^1$, $R^2$, $R^5$, and n are each as defined above,
and then the protected aminoalcohol compound represented by Formula (6b) is reacted with a cyanating agent to afford a nitrile compound (7) or a salt thereof.

The use of a protected aminoaldehyde compound represented by Formula (6aX)

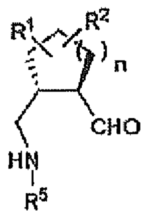

(6aX)

wherein $R^1$, $R^2$, $R^5$, and n are each as defined above as the protected aminoaldehyde compound represented by Formula (6a) makes it possible to produce a protected aminoalcohol compound represented by Formula (6bX)

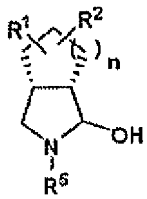

(6bX)

wherein $R^1$, $R^2$, $R^5$, and n are each as defined above. In this reaction, isomerization occurs at the α-position with respect to the carbonyl of the protected aminoaldehyde compound represented by Formula (6aX). In inducing such isomerization, the base with which the protected aminoaldehyde compound represented by Formula (6aX) is treated is particularly preferably 1,8-diazabicyclo[5.4.0]undec-7-ene or a metal alkoxide.

While in the case of producing a protected aminoalcohol compound represented by Formula (6bX) from a protected aminoaldehyde compound represented by Formula (6aX), stereoinversion at the α-position of the formyl group of the protected aminoaldehyde compound represented by Formula (6aX) is required, the use of 1,8-diazabicyclo[5.4.0]undec-7-ene or a metal alkoxide as a base efficiently promotes the stereoinversion (isomerization) and cyclization, so that the protected aminoalcohol compound represented by Formula (6bX) is obtained in a high yield with suppression of the generation of impurities. Moreover, in the case of using a protected aminoaldehyde compound in which $R^5$ is an arylmethyl such as benzyl, it is preferred to use a metal alkoxide as the base, and the use of sodium methoxide is more preferred.

Furthermore, a compound represented by Formula (7X)

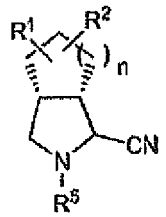

(7X)

wherein $R^1$, $R^2$, $R^5$, and n are each as defined above, is obtained as the nitrile compound represented by Formula (7) by a reaction of the protected aminoalcohol compound represented by Formula (6bX) with a cyanating agent, and from this compound represented by Formula (7X), a compound represented by Formula (8X)

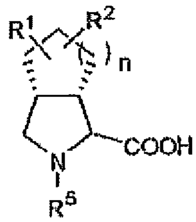

(8X)

wherein $R^1$, $R^2$, $R^5$, and n are each as defined above, or a salt thereof is obtained as the protected amino acid compound represented by Formula (8) or a salt thereof.

While the protected aminoacetal compound represented by Formula (6) or a salt thereof is treated with an acid first, the treatment can be conducted in an ordinary method by using an acid that is used for the deprotection of an acetal. Specific examples of the acid include hydrochloric acid, sulfuric acid, phosphoric acid, nitric acid, methanesulfonic acid, and p-toluenesulfonic acid.

Examples of the base to be used for the base treatment of the aldehyde resulting from the deprotection include alkali metal hydroxides (sodium hydroxide, potassium hydroxide, and the like), alkali metal carbonates (sodium carbonate, potassium carbonate, and the like), alkali metal hydrides (sodium hydride and the like), and amines (1,8-diazabicyclo[5.4.0]undec-7-ene (DBU), 1,5-diazabicyclo 4.3.0]non-5-ene (DBN), and the like). The amount of the base used is usually 0.1 mol to 20 mol, preferably 0.5 mol to 10 mol, and more preferably 1 mol to 5 mol per 1 mol of the protected acetal compound (6) or a salt thereof. There can be used a reaction solvent that is the same as that to be used in Step A, and specific examples thereof include an alcohol, an ether, a sulfoxide, an amide, a halogenated hydrocarbon, water, or a mixture thereof. The reaction temperature is usually -20°C to 60°C, preferably 0°C to 40°C. The reaction time may be chosen appropriately by checking the process of the reaction and the amount of by-products, and it is usually 30 minutes to 40 hours, preferably 1 hour to 20 hours. After the completion of the reaction, post treatment is conducted in accordance with an ordinary method. In this reaction, the steric configuration of the $\alpha$-position carbon of the aldehyde can be reversed, as required.

Subsequently, the hydroxy of the product is converted into cyano. As the cyanating agent, there is usually used one to be used for converting hydroxy into cyano, and for example, TMSCN (trimethylsilyl cyanide) can be used.

The nitrile compound (7) can also be converted into a salt thereof in accordance with an ordinary method. Examples of the salt include inorganic acid salts (a hydrochloride, a sulfate, a phosphate, a nitrate, and the like) and organic acid salts (an acetate, a citrate, a p-toluenesulfonate, a methanesulfonate, and the like).

[Step F]

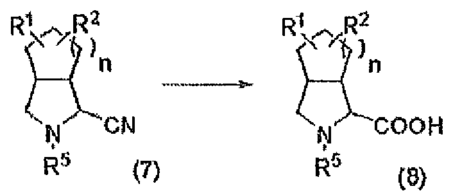

wherein $R^1$, $R^2$, $R^5$, and n are each as defined above.

In Step F, a protected amino acid compound (8) or a salt thereof is produced by hydrolyzing the nitrile compound (7) or a salt thereof.

The hydrolysis treatment of the nitrile compound (7) can be conducted in accordance with an ordinary method, and for example, it is conducted by treating the nitrile compound (7) with an acid. Examples of the acid to be used include hydrochloric acid, sulfuric acid, phosphoric acid, nitric acid, methanesulfonic, acid, and p-toluenesulfonic acid. Examples of the solvent to be used for the acid treatment include an alcohol, an ether, a sulfoxide, an amide, water, or a mixture thereof, such as those mentioned above.

The protected amino acid compound (8) can also be converted into a salt thereof in accordance with an ordinary method. Examples of the salt include inorganic acid salts (a hydrochloride, a sulfate, a phosphate, a nitrate, and the like) and organic acid salts (an acetate, a citrate, a p-toluenesulfonate, a methanesulfonate, and the like).

[Step G]

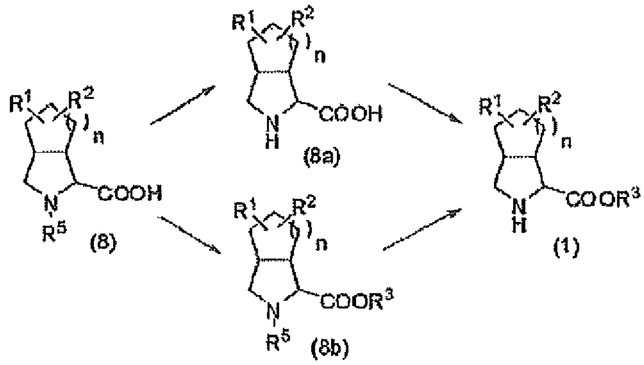

wherein $R^1$, $R^2$, $R^3$, $R^5$, and n are each as defined above.

In Step G, an ester compound (1) or a salt thereof is produced by substituting the group represented by $R^5$ into a hydrogen atom and protecting a carboxyl group.

Step G involves an operation to substitute the group represented by $R^5$ into a hydrogen atom (deprotection operation) and an operation to protect carboxyl (protection operation). The order of the two operations in Step G is not particularly limited; it is permitted to substitute the group represented by $R^5$ into a hydrogen atom and then protect a carboxyl group, and it is also permitted to protect a carboxyl group and then substitute the group represented by $R^5$ into a hydrogen atom. In the case where $R^5$ is a urethane type protective group or acyl, the protective group of $R^5$ leaves (that is, the group represented by $R^5$ is substituted into a hydrogen atom) concurrently with the hydrolysis of nitrile when treating the nitrile compound (7) with an acid in Step F. In this case, an ester compound (1) or a salt thereof is produced by protecting the carboxyl of an amino acid compound (8a) obtained as a result of the occurrence of the hydrolysis of nitrile and the leaving of the protective group of $R^5$ due to the acid treatment of the nitrile compound (7).

In Step G, in the case of conducting the deprotection operation first, the amino acid compound (8a) or a salt thereof is produced. The carboxyl group of the amino acid compound (8a) produced or a salt thereof can be protected with an ester type protective group in accordance with an ordinary method. For example, a protective group can be selected and introduced with reference to "Protective Groups in Organic Synthesis", by T. W. Greene and P. G. M. Wuts (published by Wiley-Interscience, 4th edition, 2006).

The ordinary method to protect with an ester type protective group is adopted also for the case of conducting the protection operation first to the protected amino acid compound (8) or a salt thereof. From the protected amino acid compound (8b) protected with an ester type protective group or a salt thereof, an ester compound (1) or a salt thereof is produced by removing the protective group of $R^5$ (substituting the group represented by $R^5$ into a hydrogen atom) by deprotection operation.

A preferred example of the protection operation to protect the carboxyl of the protected amino acid compound (8) or a salt thereof with tert-butyl is provided here. The tert-butyl protection of the carboxyl can be conducted by bringing the protected amino acid compound (8) or a salt thereof into contact with di-tert-butyl dicarbonate in the presence of a tertiary amine, preferably N,N-dimethylaminopyridine. The amount of the tertiary amine used is usually 0.01 to 0.5 mol, preferably 0.02 to 0.1 mol per 1 mol of the protected amino acid compound (8) or a salt thereof, and the amount of di-tert-butyl dicarbonate used is usually within the range of from 1 to 10 mol, preferably from 1.5 to 3 mol per 1 mol of the protected amino acid compound (8) or a salt thereof. While this reaction can be conducted either in the presence or in the absence of a solvent, it is preferred to conduct it in the presence of a solvent. As such a solvent, there can be used

tert-butyl methyl ether or the like, and the amount thereof used is usually 3 to 50 L, preferably 5 to 20 L per 1 kg of the protected amino acid compound (8) or a salt thereof. In this reaction, it is preferred to mix tert-butyl alcohol in order to increase the yield or the reaction rate. The amount thereof used is usually 1 to 10 mol, preferably 1. 5 mol to 5 mol per 1 mol of the protected amino acid compound (8).

The reaction temperature of this reaction is usually 0 to 80°C, preferably 20 to 50°C, and the reaction time is usually 2 to 48 hours, preferably 8 to 32 hours.

Moreover, the ester compound (1) can be converted into a salt thereof in accordance with an ordinary method, as required. Examples of the salt include inorganic acid salts (a hydrochloride, a sulfate, a phosphate, a nitrate, and the like) and organic acid salts (an acetate, a citrate, a p-toluenesulfonate, a methanesulfonate, and the like), and preferred is a hydrochloride.

In each of the above described Step A through Step F, the product may be purified, as required. The purification may be conducted in an ordinary method; for example, recrystallization, extraction, and column chromatography can be used.

EXAMPLES

The present invention is described in more detail below with reference to Examples.

Production Example 1 (Synthesis of aldehyde compound (2))

To 1.5 L of chloroform were added 100.0 g (0.846 mol) of 1,6-hexanediol, 40.3 g (0.338 mol) of potassium bromide, 106.6 g (1.269 mol) of sodium hydrogen carbonate, and 0.46 g (0.0030 mol) of TEMPO, followed by stirring. The resulting mixture was cooled to a temperature of 10°C or lower and 1227.5 g of a 11.8% aqueous sodium hypochlorite solution was dropped over 3 hours and 40 minutes with a temperature of 2 to 9°C maintained, followed by stirring at about 5°C for 1 hour. To this solution was added 21.0 g (0.0846 mol) of sodium thiosulfate pentahydrate, followed by stirring for 15 minutes, and then the precipitated solid was removed by filtration. The organic layer of the two-layer solution resulting from the removal of the solid by filtration was separated. To the remaining aqueous layer was added 500 mL of chloroform, followed by stirring at room temperature for 20 minutes, and then the organic layer was separated. The two organic layers were combined, and to the resultant organic layer (2898.3 g) were added 0.72 g (0.0118 mol) of acetic acid and 1.01 g (0.0118 mol) of piperidine. This solution was heated to 40 to 45°C and stirred for 12 hours. After cooling to near room temperature, 226 g of 20% brine was added, followed by stirring at 20 to 30°C for 10 minutes, and then the organic layer (2890.6 g) was separated. The residue (229.9 g) obtained by concentrating the organic layer under reduced pressure was subjected to vacuum distillation (degree of vacuum: 8 to 14 Torr, warm water bath temperature: 40 to 70°C), affording 44.7 g of 1-cyclopentene-1-carboaldehyde.

Example 1 (Step A)

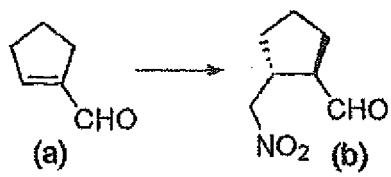

To a solution of 9.5 g (156.0 mmol) of nitromethane, 1.7 g (5.2 mmol) of (S)-2-(diphenyltrimethylsilyloxymethyl)proline (a pyrrolidine compound), and 0.13 g (1.04 mmol) of benzoic acid in 2-propanol (104 mL) was added 4.0 g (41.6 mmol) of 1-formylcyclopentene (a) (aldehyde compound (2)) over 5 hours. The reaction mixture was stirred at the same temperature. After confirmation of the completion of the reaction by gas chromatography, the solvent was evaporated under reduced pressure, and then the residue was purified with silica gel chromatography (ethyl acetate : toluene = 1: 80), affording 7.3 g (yield: 88.9%) of Compound (b) (nitroaldehyde compound (3)) as a pale yellow oil.

[GC analysis conditions]

Capillary column: Agilent J&W DB-WAX, 30 m × 0.53 mm, 1.00 μm
Carrier gas: helium; flow rate: 20 mL/minute; injection of the entire amount/the amount of injection 1 μL
Column temperature: 70°C (5 minutes) → 10°C/minute → 200°C (12 minutes)

Vaporization chamber temperature: 250°C; detector (FID) temperature: 250°C
Retention time
1-Formylcyclopentene (a): 6.1 minutes

Compound (b): 17.4 minutes
$^1$H-NMR($CDCl_3$)δ: 9.67(1H, d, J=1.7 Hz), 4.47-4.37(2H, m), 3.06-2.97(1H, m), 2.67-2.61(1H, m), 2.05-1.90(3H, m), 1.83-1.73(1H, m), 1.71-1.60(1H, m), 1.51-1.41(1H, m) $^{13}$C-NMR($CDCl_3$)δ: 201.2, 78.5, 54.6, 37.8, 30.2, 26.6, 24.3

Example 2 (Step A)

To a solution of 1.9 g (31.2 mmol, 3.0-fold mol) of nitromethane, 0.068 g (0.21 mmol, 0.02-fold mol) of (S)-2-(diphenyltrimethylsilyloxymethyl)proline, and 0.003 g (0.05 mmol, 0.005-fold mol) of acetic acid in 2-propanol (3.0 mL) was dropped 1.0 g (10.4 mmol) of 1-formylcyclopentene (a) over about 5 minutes. A reaction was conducted for 24 hours, followed by an analysis conducted under the GC analysis conditions of Example 1, which revealed that the residual feed was 63.7%.

[Calculation formula of residual feed]

Residual feed % = [GC area of 1-formylcyclopentene (a)]/([GC area of 1-formylcyclopentene (a)] + [GC area of Compound (b)]) × 100

Example 3 (Step A)

To a solution of 0.63 g (10.4 mmol, 2.0-fold mol) of nitromethane, 0.034 g (0.10 mmol, 0.02-fold mol) of (S)-2-(diphenyltrimethylsilyloxymethyl)proline (a pyrrolidine compound), and 0. 0016 g (0.03 mmol, 0.005-fold mol) of acetic acid in 2-propanol (1.5 mL) was added 9.4 mL (0.52 mmol, 0.1-fold mol) of water, followed by the dropping of 0.5 g (5.2 mmol) of 1-formylcyclopentene (a) over about 5 minutes. A reaction was conducted for 25 hours, followed by an analysis conducted under the GC analysis conditions of Example 1, which revealed that the residual feed was 15.8%. A quantitative GC yield analysis conducted under the GC analysis conditions of Example 1 revealed that the yield of Compound (b) was 74.8%.

Examples 4 to 8 (Step A)

A reaction was conducted under the same conditions as in Example 3 except for changing the amounts of 2-propanol and water as shown in the following Table 1.

[Table 1]

| | Isopropanol | Water (-fold mol) |
|---|---|---|
| Example 4 | 1.5 mL | 94 μL (1.0-fold mol) |
| Example 5 | 1.5 mL | 0.19 mL (2.0-fold mol) |
| Example 6 | 1.2 mL | 0.3 mL (3.2-fold mol) |
| Example 7 | 1.0 mL | 0.5 mL (5.3-fold mol) |
| Example 8 | 0.75 mL | 0.75 mL (8.0-fold mol) |

In Table 2 are shown the amount of the residual feed at 20 to 25 hours from the start of the reaction, the amount of the residual feed at the quantitative yield determination, and the quantitative GC yield of Compound (b).

[Table 2]

| | Reaction time | Residual feed | Residual feed at quantitative yield determination | Yield of Compound (b) |
|---|---|---|---|---|
| Example 4 | 25 hours | 0.90% | 0.40% | 78.90% |
| Example 5 | 25 hours | 0.30% | 0.20% | 80.20% |

(continued)

| | Reaction time | Residual feed | Residual feed at quantitative yield determination | Yield of Compound (b) |
|---|---|---|---|---|
| Example 6 | 20 hours | 0.50% | 0.20% | 78.30% |
| Example 7 | 20 hours | 1.40% | 0.50% | 76.10% |
| Example 8 | 20 hours | 2.10% | 0.70% | 73.60% |

Examples 9 to 12 (Step A)

A reaction was conducted under the same conditions as in Example 3 except for exchanging 2-propanol used in Example 3 into methanol and changing the amounts of methanol and water as shown in the following Table 3.

[Table 3]

| | Methanol | Water (-fold mol) |
|---|---|---|
| Example 9 | 1.4 mL | 0.10 mL (1.1-fold mol) |
| Example 10 | 1.35 mL | 0.15 mL (1.6-fold mol) |
| Example 11 | 1.20 mL | 0.30 mL (3.2-fold mol) |
| Example 12 | 1.00 mL | 0.50 mL (5.3-fold mol) |

In Table 4 are shown the amount of the residual feed at 18 to 22 hours from the start of the reaction, the amount of the residual feed at the quantitative yield determination, and the quantitative GC yield of Compound (b).

[Table 4]

| | Reaction time | Residual feed | Residual feed at quantitative yield determination | Yield of Compound (b) |
|---|---|---|---|---|
| Example 9 | 22 hours | 0.10% | 0.10% | 91.80% |
| Example 10 | 18 hours | 0.10% | 0.10% | 89.30% |
| Example 11 | 18 hours | 0.30% | 0.30% | 89.60% |
| Example 12 | 18 hours | 0.80% | 0.70% | 84.30% |

Example 13 (Step A)

To a solution of 3.05 g (49.9 mmol, 1.2-fold mol) of nitromethane, 0.27 g (0.83 mmol, 0.02-fold mol) of (S)-2-(diphenyltrimethylsilyloxymethyl)proline (a pyrrolidine compound), and 0.013g (0.21 mol, 0.005-fold mol) of acetic acid in methanol (8.8 g) was added 1.2 g (66.6 mmol, 1.6-fold mol) of water, followed by the dropping of 4.0 g (41.6 mmol) of 1-formylcyclopentene (a) over 2.5 hours. A reaction was conducted for 6 hours, followed by an analysis conducted under the GC analysis conditions of Example 1, which revealed that the residual feed was 0.8%. Subsequently, after leaving at rest for 14 hours, a quantitative GC yield analysis conducted under the GC reaction analysis conditions of Example 1 revealed that the yield of Compound (b) was 84.4%.

The analysis of Compound (b) under the following GC optical analysis conditions failed to observe the enantiomer of Compound (b).

[GC optical analysis conditions]

Capillary column: Astec CHIRALDEX β-DM, 30 m × 0.25 mm, 0.12 μm

Carrier gas: helium 10 ml/min; flow rate: 1.13 mL/minute Split ratio 50: 1 / the amount of injection 1 μL

Column temperature: 100°C (0 minutes) → 3°C/minute → 200°C (10 minutes)

Vaporization chamber temperature: 200°C; detector (FID) temperature: 250°C

Retention time

Compound (b): 16.8 minutes

Compound (b) enantiomer: 17.1 minutes.

Example 14 (Step A)

To a solution of 4.57 g (74.9 mmol, 1.2-fold mol) of nitromethane, 0.31 g (0.94 mmol, 0.015-fold mol) of (S)-2-(diphenyltrimethylsilyloxymethyl)proline (a pyrrolidine compound), and 0.011 g (0.19 mmol, 0.003-fold mol) of acetic acid in methanol (12.9 g) was added 1.8 g (99.9 mmol, 1.6-fold mol) of water, followed by the dropping of 6.0 g (62.4 mmol) of 1-formylcyclopentene (a) over 4 hours. A reaction was conducted for 3 hours, followed by an analysis conducted under the GC analysis conditions of Example 1, which revealed that the residual feed was 14.8%. Subsequently, after leaving at rest for 14 hours, a quantitative GC yield analysis conducted under the GC reaction analysis conditions of Example 1 revealed that the residual feed was 1.0% and the yield of Compound (b) was 84.4%.

The analysis of Compound (b) under the GC optical analysis conditions described in the section of Example 13 revealed that its optical purity was 99.9%e.e.

Example 15 (Step B)

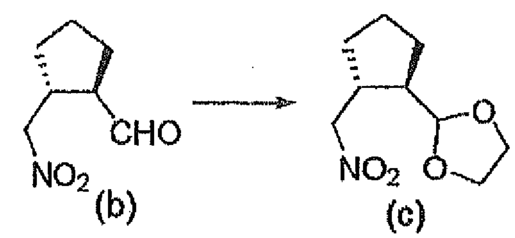

(b) (c)

A solution of 13.0 g (82.7 mmol) of Compound (b), 6.0 g (95.2 mmol) of ethylene glycol, and 0.267 g of p-toluenesulfonic acid in toluene (315 mL) was refluxed for 2 hours while water generated was removed with a Dean-Stark trap. After confirming the complete consumption of the starting materials by conducting an analysis under the GC analysis conditions described in Example 1, the resulting mixture was cooled and then the solvent was evaporated under reduced pressure. The residue was purified with column chromatography (ethyl acetate : toluene = 1: 80), affording 15.3 g (yield: 92%) of nitroacetal (c) (nitroacetal compound (4)) as a colorless oil. $^1$H-NMR ($CDCl_3$)δ: 4.78(1H, d, J=4.9 Hz), 4.70(1H, dd, J=12.2, 4.9 Hz), 4.25(1H, dd, 12.2, 9.8 Hz), 4.00-3.92(2H, m), 3.91-3.82(2H, m), 2.66-2.60(1H, m)1.98-1.81(3H, m), 1.71-1.58(3H, m), 1.47-1.38(1H, m)

$^{13}$C-NMR($CDCl_3$)δ: 106.3, 80.0, 65.1, 64.9, 45.9, 39.2, 32.0, 27.9, 24.4

Example 16 (Step B)

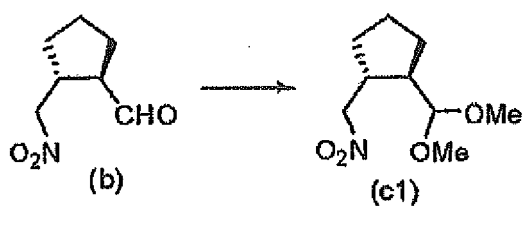

(b) (c1)

In methanol (89 mL) containing 9.4 g (89 mmol) of trimethoxymethane and 0.164 g of p-toluenesulfonic acid was dissolved 7.0 g (44.5 mmol) of Compound (b), and the resulting solution was stirred at 25°C for 24 hours. The reaction mixture was neutralized with the addition of sodium methoxide/methanol, and methanol was evaporated under reduced pressure from the neutralized reaction mixture. The residue was dissolved in methyl tert-butyl ether (45 mL), and the resulting ether solution was washed with water (35 mL) three times. The water-washed ether layer was dried over magnesium sulfate and then the solvent was evaporated under reduced pressure. The residue was purified with column chromatography (ethyl acetate : toluene = 1: 80), affording 7.2 g (yield: 80%) of nitroacetal (c1) (nitroacetal compound (4)) as a colorless oil. $^1$H-NMR($CDCl_3$)δ: 4.67(1H, dd, J=12.2, 5.4 Hz), 4.25-4.19(2H, m)3.38(3H, s), 33.32(3H, s), 2.56-2.47(1H, m), 2.00-1.86(2H, m)1.84-1.75(1H, m)1.67-1.60(2H, m), 1.58-1.48(1H, m), 1.46-1.37(1H, m)

$^{13}$C-NMR($CDCl_3$)δ: 107.7, 79.9, 55.3, 52.4, 45.1, 40.3, 30.9, 27.9, 24.0

Example 17 (Step C)

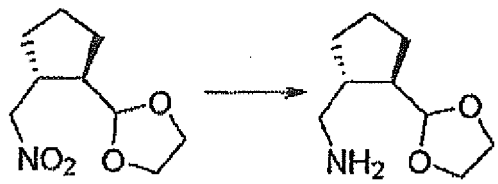

(c) (d)

Under a hydrogen atmosphere (500 kPa), a mixture of 15.1 g (75.0 mmol) of nitroacetal (c) and 5.0 g of 20% palladium hydroxide/carbon was stirred in methanol (75 mL) at room temperature (about 27°C) for 2.5 hours. The reaction mixture was filtered under a nitrogen atmosphere and the solvent was evaporated to dryness, affording a colorless residue. The resulting unpurified aminoacetal (d) (aminoacetal compound (5)) in an amount of 12.3 g (yield: 96%) was subjected to the subsequent step because a $^{1}$H-NMR analysis confirmed it to be sufficiently pure.
$^{1}$H-NMR($CDCl_3$)$\delta$: 4.75(1H, d, J=4.9 Hz), 4.00-3.94(2H, m), 3.87-3.82(2H, m)2.78(1H, dd, J=12.2, 5.9 Hz), 2.59(1H, dd, J=12.2, 7.3 Hz), 1.96-1.71(4H, m), 1.61-1.54(5H, m), 1.35-1.27(1H, m)
$^{13}$C-NMR($CDCl_3$)$\delta$: 107.1, 64.9, 64.7, 47.1, 46.4, 44.3, 31.0, 28.1, 24.9

Example 18 (Step C)

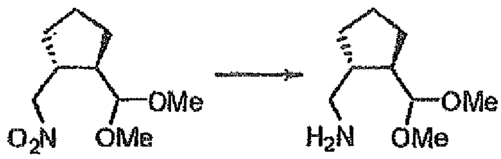

(c1) (d1)

A mixture prepared by mixing 6.3 g (31.0 mmol) of nitroacetal (c1) and 2.1 g of 20% $Pd(OH)_2$/carbon with methanol (31 mL) was stirred under a nitrogen overpressure (500 kPa) at room temperature (about 27°C) for 2.5 hours. The insoluble matter was removed by filtration under a nitrogen atmosphere and the solvent was evaporated from the filtrate under reduced pressure, affording a colorless residue. The resulting unpurified aminoacetal (d1) (aminoacetal compound (5)) in an amount of 5.2 g (yield: 96%) was subjected to the subsequent step because a $^{1}$H-NMR analysis confirmed it to be sufficiently pure.
$^{1}$H-NMR($CHCl_3$)$\delta$: 4.14(1H, d, J=7.3 Hz), 3.37(3H, s), 3.32(3H, s), 2.78(1H, dd, J=12.7, 5.86 Hz), 2.55(1H, dd, J=12.7, 7.32 Hz), 1.92-1.62(6H, m), 1.58-1.47(3H, m), 1.37-1.29(1H, m) $^{13}$C-NMR($CDCl_3$)$\delta$: 107.8, 54.5, 52.1, 46.9, 45.1, 44.8, 30.7, 28.1, 24.5

Example 19 (Step D)

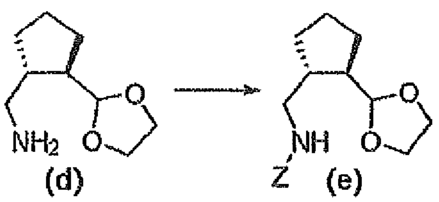

(d) (e)

A solution of 12.3 g (71.8 mmol; unpurified) in a mixture of water (200 mL) and methyl tert-butyl ether (150 mL) was cooled to 0°C and then 31.1 g (225.0 mmol) of potassium carbonate was added, followed by stirring. Into the mixture

was added 14.1 g (82.5 mmol) of benzyloxycarbonyl chloride over 10 minutes, and the resulting mixture was heated to room temperature and stirred for 12 hours. The stirring of the mixture was stopped, the organic layer separated was taken, and the aqueous layer was subjected to extraction with 50 mL of methyl tert-butyl ether three times. These organic layers were combined and dried over magnesium sulfate, and then the solvent was evaporated under reduced pressure. The resulting residue was purified with silica gel column chromatography (ethyl acetate: heptane = 1 : 3), affording 21.2 g (yield: 97%) of the objective protected acetal (e) (protected aminoacetal compound (6)) as a colorless oil.
$^1$H-NMR($CDCl_3$) $\delta$: 7.37-7.29 (5H, m), 5.6 (1H, brs), 5.13-5.06 (2H, m), 4.72 (1H, d, J=4.9 Hz), 3.96-3.94 (2H, m), 3.86-3.83 (2H, m), 3.31-3.25 (1H, m), 3.11-3.04 (1H, m), 2.04-1.99 (1H, m), 1.87-1.73(3H, m), 1.61-1.56 (3H, m), 1.35-1.28 (1H, m) $^{13}$C-NMR($CDCl_3$) $\delta$: 15.6, 137.0, 128.4, 127.9, 127.8, 106.7, 66.3, 64.9, 64.8, 47.0, 45.9, 40.0, 31.6, 28.9, 24.9

Example 20 (Step D)

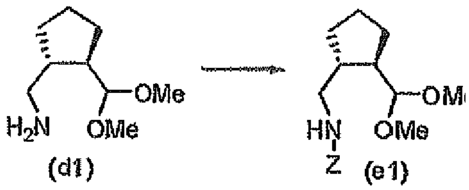

(d1) (e1)

A solution of 5.0 g (28.9 mmol; unpurified) of aminoacetal (d1 in a mixture of water (80 mL) and methyl tert-butyl ether (60 mL) was cooled to 0°C and then 12. 0 g (86.7 mmol) of potassium carbonate was added, followed by stirring. Into the mixture was added 5. 9 g (34.7 mmol) of benzyloxycarbonyl chloride over 10 minutes, and the resulting mixture was heated to room temperature and stirred for 18 hours. The stirring of the mixture was stopped, the organic layer separated was taken, and the aqueous layer was subjected to extraction with 50 mL of methyl tert-butyl ether three times. These organic layers were combined and dried over magnesium sulfate, and then the solvent was evaporated under reduced pressure. The resulting residue was purified with silica gel column chromatography (ethyl acetate : heptane = 1 : 3), affording 8.6 g (yield: 97%) of the objective protected acetal (e1) (protected aminoacetal compound (6)) as a colorless oil.
$^1$H-NMR($CDCl_3$) $\delta$: 7.36-7.27 (5H, m), 5.67 (1H, s), 5.13-5.06 (2H, m), 4.12(1H, d, J=6.8 Hz), 3.39 (3H, s), 3.30 (3H, s), 3.27-3.21 (1H, m), 3.08-3.01 (1H, m), 1.96-1.89 (2H, m), 182-1.70 (2H, m), 1.58-1.45(3H, m), 1.34-1.28 (1H, m) $^{13}$C-NMR($CDCl_3$) $\delta$: 156.6, 136.9, 128.4, 127.9, 127.8, 107.8, 66.2, 65.1, 55.4, 52.0, 46.0, 40.8, 31.5, 28.8, 24.8

Example 21 (Step D)

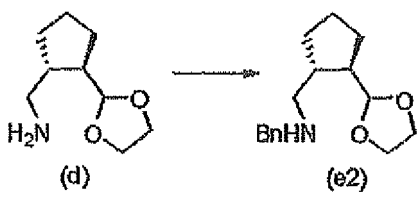

(d) (e2)

Into a flask equipped with a Dean-Stark trap was charged a solution prepared by dissolving 9.9 g (57.7 mmol) of aminoacetal (d) in toluene (115 mL), followed by the addition of 6.4 g (60.6 mmol) of benzaldehyde. The resulting mixture was stirred at 120°C for 2 hours with azeotropic removal of water. After the amount of the removed water reached a theoretical amount, the solvent was evaporated under vacuum and then the residue was dissolved in methanol (115 mL). Subsequently, 3.3 g (86.5 mmol) of sodium borohydride was added slowly with cooling in an ice-bath, and after the addition, the temperature was raised to room temperature, followed by stirring at the same temperature for 2 hours. Under vacuum, methanol was evaporated and the residue was dissolved in ethyl acetate (88 mL). This solution was washed with water (70 mL) three times. The washed ethyl acetate layer was dried over magnesium sulfate and then the solvent was evaporated under reduced pressure. The resulting residue was purified with silica gel column chromatography (ethyl acetate : heptane = 1 : 3), affording 10.8 g (yield: 72%) of the objective protected acetal (e2) (protected aminoacetal compound (6)) as a colorless oil. $^1$H-NMR($CDCl_3$) $\delta$: 7.33-7.22 (5H, m), 4.76 (1H, d, J=4.9 Hz), 3.98-3.91(2H, m),

3.86-3.78 (4H, m), 2.70 (1H, dd, J=11.2, 6.3 Hz), 2.55 (1H, dd, J=11.2, 7.3 Hz), 2.12-2.03 (1H, m), 1.92-1.69 (4H, m), 1.61-1.52 (3H, m), 1.36-1.25 (1H, m) $^{13}$C-NMR($CDCl_3$) δ: 140.8, 128.2, 128.0, 126.6, 107.1, 65.0, 64.8, 54.8, 54.0, 47.1, 40.9, 31.9, 28.0, 25.0

Example 22 (Step D)

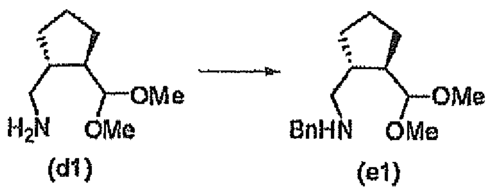

(d1) (e1)

Into a flask equipped with a Dean-Stark trap were charged 10.0 g (57.7 mmol) of aminoacetal (d1) and toluene (115 mL), followed by adding 6.4 g (60.6 mmol) of benzaldehyde thereto. This mixture was azeotropically dehydrated for 2 hours until a theoretical amount of water flowed out. Subsequently, the solvent was evaporated under vacuum and methanol (115mL) was added to the residue. The mixture was cooled in an ice bath and 3.3 g (86.6 mmol) of sodium borohydride was added in small portions. After the completion of the addition, the temperature was raised to room temperature, followed by stirring at this temperature for 2 hours. After the reaction, methanol was evaporated under vacuum, and ethyl acetate (88 mL) was added to the residue, which was then dissolved therein. The resulting ethyl acetate solution was washed with 70 mL of water three times. The washed ethyl acetate solution was dried over magnesium sulfate, and then the solvent was evaporated under reduced pressure. The resulting residue was purified with silica gel column chromatography (ethyl acetate : heptane = 1 : 3), affording 10.9 g (yield: 72%) of the objective protected acetal (e1) (protected aminoacetal compound (6) as a colorless oil. The $^1$H-NMR data was the same as that given above.
$^1$H-NMR($CDCl_3$) δ: 7.32-7.21 (5H, m), 4.14 (1H, d, J=7.3 Hz), 3.79(2H, s), 3.34(3H, s), 3.30(3H, s), 2.69 (1H, dd, J=11.2, 6.3 Hz), 2.51 (1H, dd, J=11.2, 7.3 Hz), 2.01-1.86(3H, m), 1.82-1.76 (1H, m), 1.72-1.66 (1H, m), 1.57-1.46 (3H, m), 1.34-1.26 (1H, m)
$^{13}$C-NMR($CDCl_3$) δ: 140.7, 128.1, 127.9, 126.6, 108.0, 54.8, 54.7, 54.0, 52.4, 46.1, 41.7, 31.7, 28.2, 24.8

Example 23 (Step E)

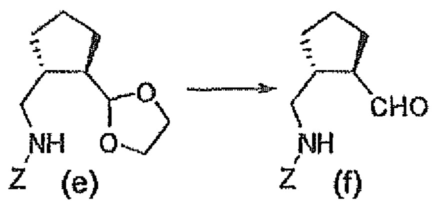

(e) (f)

To a solution of 21.2 g (69.4 mmol) of protected acetal (e) in THF (278 mL) was added 278 mL (1112 mmol) of 4 M hydrochloric acid, followed by stirring at room temperature. After 20 hours, solid potassium carbonate was added carefully to adjust pH to 8. The resulting mixture was subjected to extraction with 70 mL of methyl tert-butyl ether three times, and the resulting organic layers were combined, dried over sodium sulfate, and then concentrated under vacuum. The resulting residue was purified with silica gel column chromatography (ethyl acetate : heptane = 1: 3), affording 14.1 g (yield: 77.8%) of the objective aldehyde (f) as a colorless oil.
$^1$H-NMR($CDCl_3$) δ: 9.62 (1H, d, J=1.5 Hz), 7.35-7.28 (5H, m),
5.15-5.05 (2H, m), 5.03 (1H, brs), 3.28-3.14 (2H, m), 2.52-2.46 (1H, m), 2.43-2.35 (1H, m), 1.92-1.80(3H, m), 1.75-1.67 (1H, m), 1.64-1.53 (1H, m), 1.40-1.31 (1H, m) $^{13}$C-NMR($CDCl_3$) δ: 203.5, 156.5, 136.5, 128.4, 128.1, 128.0, 66.6, 55.8, 45.0, 40.7, 30.3, 26.7, 24.7

Example 24 (Step E)

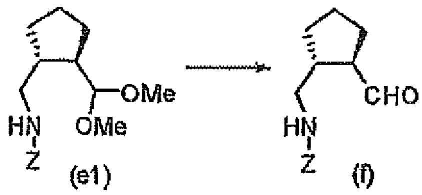

To a solution of 8.0 g (26.0 mmol) of protected acetal (e1) in THF (278 mL) was added 278 mL (1112 mmol) of 4 M hydrochloric acid, followed by stirring at room temperature. After 20 hours, solid potassium carbonate was added carefully to adjust pH to 8. The resulting mixture was subjected to extraction with 70 mL of methyl tert-butyl ether three times, and the resulting organic layers were combined, dried over sodium sulfate, and then concentrated under vacuum. The resulting residue was purified with silica gel column chromatography (ethyl acetate: heptane = 1: 3), affording 5.1 g (yield: 75%) of the objective aldehyde (f) as a colorless oil. The $^1$H-NMR data was the same as that given above.

Example 25 (Step E)

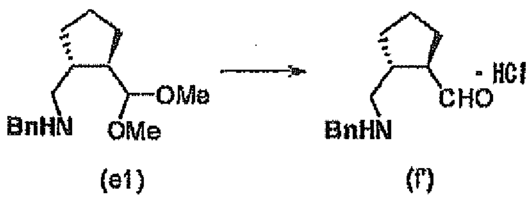

To a solution of 2.2 g (7.6 mmol) of protected acetal (e1) in IPA (7.6 mL) were added water (15mL) and 35% hydrochloric acid (1.3 mL), 15.2 mmol), and the resulting mixture was stirred at room temperature for 2 hours. After confirmation of the completion of the reaction by HPLC analysis, toluene (15mL) was added, followed by stirring for 0.5 hours. The aqueous layer was taken out by separation, and the aqueous layer was subjected to extraction with 15 mL of toluene twice. The organic layers were combined and then concentrated under vacuum, affording 1. 9 g (yield: 98%) of the objective aldehyde hydrochloride (f') as a yellow oil.
$^1$H-NMR($CDCl_3$) δ: 8.46 (1H, s), 7.35-7.29 (5H, m), 4.94-4.85(2H, m), 4.12-4.09 (1H, m), 3.80-3.70 (1H, m), 3.62-3.59 (1H, m), 2.98-2.89 (1H, m), 1.85-1.58 (3H, m), 1.55-1.43 (1H, m), 1.32-1.12 (2H, m)
$^{13}$C-NMR($CDCl_3$) δ: 183.7, 132.0, 131.6, 131.3, 131.2, 66.8, 59.3, 55.4, 39.5, 34.9, 30.2, 26.7

Example 26 (Step E)

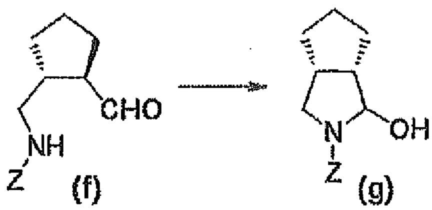

To a solution of 13.08 g (50.0 mmol) of aldehyde (f) in methanol (100mL) was added 9.1 g (60.0 mmol) of DBU at room temperature, followed by stirring for 22 hours. The solvent was evaporated under reduced pressure, and the resulting residue was purified with silica gel column chromatography (ethyl acetate : heptane = 1 : 3), affording 8.12 g (yield: 62%) of the objective hemiaminal (g) as a colorless oil.
$^1$H-NMR($CDCl_3$) δ: 7.39-7.29 (5H, m), 5.31-5.28 (1H, m), 5.17-5.10 (2H, m), 3.77-3.72(2H, m), 3.26-3.19 (1H, m), 2.89-2.79 (1H, m), 2.57-2.51 (1H, m), 1.93-1.77(2H, m), 1.68-1.43 (4H, m)

Example 27 (Step E)

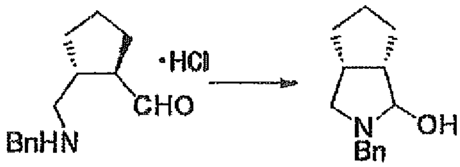

(f') (g')

To a solution of 1.8 g (7.1 mmol) of aldehyde hydrochloride (f') in methanol (15 mL) was dropped 1.6 g (21.3 mmol) of a 70% sodium methoxide/methanol solution over 10 minutes. This mixture was stirred for 12 hours and then added to ice water (15 mL). The resulting mixture was subjected to extraction with 15 mL of methyl tert-butyl ether three times, and the resulting organic layers were combined, dried over sodium sulfate, and then concentrated under vacuum. The solvent was evaporated under reduced pressure, affording 1.4 g (yield: 91%) of the objective hemiaminal (g) as a pale yellow oil. The $^1$H-NMR data was the same as that given above. $^1$H-NMR($CDCl_3$) $\delta$: 7.36-7.21 (5H, m), 3.92-3.73 (3H, m), 3.00-2.96 (1H, m), 2.68-2.58 (1H, m), 2.55-2.45 (1H, m), 2.43-2.36 (1H, m), 1.82-1.62 (3H, m) 1.49-1.36 (3H, m)

Example 28 (Step E)

Synthesis of (1S,3aR,6aS)-octahydrocyclopenta[c]pyrrole-1-carbonitrile

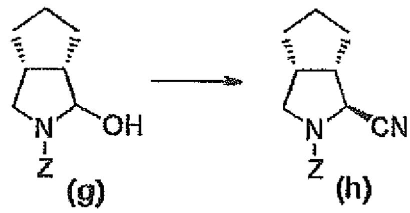

(g) (h)

Under a nitrogen atmosphere, 3.9 g (4.9 mL; 39.2 mmol) of TMSCN was added to 100.0 g (19.1 mmol) of a 5 wt% hemiaminal (g)/toluene solution at -40°C. Subsequently, a solution of 25.6 g (5.0 mL; 39.2 mmol) of $BF_3$•OEt in toluene (28 mL) was added over 10 minutes. The mixture was stirred at -40°C for 1.5 hours and then warmed slowly to 0°C. Water (10mL) was added and then a 35% aqueous potassium hydroxide solution (19.6 mL) was added, followed by stirring for 30 minutes with pH adjusted to between 12 and 13. Subsequently, 17.8 g (15 mL; 28.7 mmol) of a 12% aqueous sodium hypochlorite solution was added and the mixture was stirred with a temperature of 0 to 5°C maintained. A solution of 1.8 g of sodium sulfate in water (16.2 mL) was dropped, and the pH was adjusted to 7 to 8 with concentrated sulfuric acid. The organic layer was separated, washed with 50 mL of water three times, and dried over magnesium sulfate. The solvent was evaporated under reduced pressure, followed by purification of respective isomers of nitrile (h) (nitrile compound (7)) with silica gel column chromatography (ethyl acetate : heptane = 1: 5), affording 3.15 g of the exo form and 1.38 g of the end form (combined yield: 88%).
Exo form: $^1$H-NMR($CDCl_3$) $\delta$: 7.38-7.30 (5H, m), 5.21-5.19 (2H, m), 4.42 (1H, d, J=32.2 Hz), 3.68-3.63 (1H, m), 3.40-3.36 (1H, m), 2.92-2.89(2H, m), 2.07-1.98 (1H, m), 1.95-1.86 (1H, m), 1.80-1.70 (1H, m), 1.66-1.55 (1H, m), 1.46-1.45 (2H, m) $^{13}$C-NMR($CDCl_3$) $\delta$: 154.0, 135.9, 128.4, 128.0, 127.8, 118.5, 67.5, 53.0, 51.8, 49.3, 41.9, 32.0, 31.9, 25.4

Example 29 (Step F and Step G)

Synthesis of

(1S,3aR,6aS)-octahydrocyclopenta[c]pyrrole-1-carboxylic acid hydrochloride

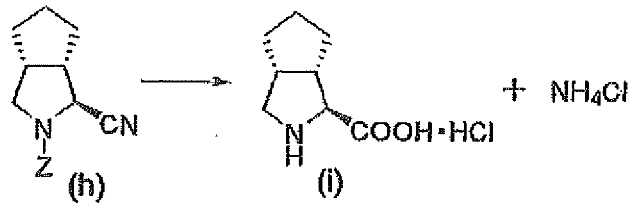

(h) (i)

To a solution of 1.2 g (4.4 mmol) of nitrile (h) in methanol (7.2 mL) was added 21.6 mL (86.4 mmol) of 4M hydrochloric acid. The mixture was refluxed for 24 hours and then cooled to about 60°C. The solvent was evaporated under reduced pressure and the resulting residue was dissolved in water (30 mL), followed by washing with 30 mL of toluene three times. Water was evaporated to dryness under reduced pressure, affording 1.01 g of a mixture of a hydrochloride of amino acid (i) and ammonium chloride as a pale brown solid.
$^1$H-NMR($D_2O$) δ: 3.79 (1H, d, J=6.8 Hz), 3.55-3.51 (1H, m), 2.85-2.81 (3H, m), 1.73-1.39 (6H, m)
$^{13}$C-NMR($D_2O$) δ: 173.3, 67.0, 52.8, 48.7, 43.6, 33.2, 32.8, 26.4 Example 30 (Step G)

Synthesis of tert-butyl

(1S,3aR,6aS)-octahydrocyclopenta[c]pyrrole-1-carboxylate

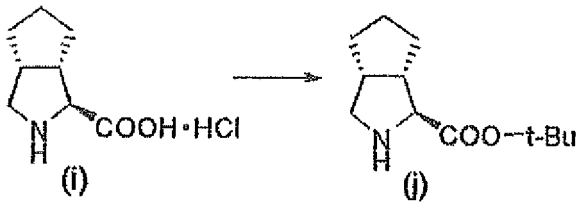

(i) (j)

[Isolation of free base]

To 160 mg of the mixture of the hydrochloride of amino acid (i) and ammonium chloride prepared in Example 29 were added 0.7 mL of chloroform and 1.0 mL of tert-butyl acetate. The resulting suspension was cooled to 0°C and then 0.15 mL of methanesulfonic acid was added. The mixture was warmed slowly to 25°C and then stirred at this temperature for 18 hours. The resulting white suspension was cooled to 0°C, and then 0.8 mL of a 50% aqueous sodium hydroxide solution was added carefully with the temperature kept at 20°C or lower. Water (10 mL) and chloroform (10 mL) were added and then stirred for 15 minutes. The layers were separated and the aqueous layer was subjected to extraction with 10 mL of chloroform three times. The organic layers were combined, dried over magnesium sulfate, and filtered. The solvent was then evaporated under reduced pressure. The residue was purified with silica gel column chromatography (ethyl acetate : methanol = 1 : 1), affording 0.022 g (0.103 mmol: overall yield from nitrile (h): 16%) of tert-butyl ester (j) (ester compound (1)) as a pale green oil. $^1$H-NMR($CDCl_3$) δ: 3.33-3.28 (1H, m), 3.15 (1H, d, J=5.9 Hz), 2.62-2.50 (2H, m), 2.48-2.44 (1H, m), 2.13 (1H, s), 1.80-1.68 (1H, m), 1.66-1.55 (5H, m), 1.47(9H, s)
$^{13}$C-NMR($CDCl_3$) δ: 173.7, 80.6, 68.1, 53.9, 49.7, 44.2, 32.3, 32.1, 28.0, 25.3

[Isolation of hydrogenoxalate]

To 250 mg of the mixture of the hydrochloride of amino acid (i) and ammonium chloride prepared in Example 29 were added 4 mL of chloroform and 1.7 mL of tert-butyl acetate. The resulting suspension was cooled to 0°C and then 0.25 mL of methanesulfonic acid was added. The mixture was warmed slowly to 25°C and then stirred at this temperature for 18 hours. The resulting white suspension was cooled to 0°C, and then 1.3 mL of a 50% aqueous sodium hydroxide solution was added carefully with the temperature kept at 20°C or lower. Water (15 mL) and chloroform (15 mL) were added and then stirred for 15 minutes. The layers were separated and the aqueous layer was subjected to extraction with 15 mL of chloroform three times. The organic layers were combined, dried over magnesium sulfate, and filtered. The solvent was then evaporated under reduced pressure. To the pale green residue was added a solution of 2.5 mL of tert-butyl acetate and 146.7 mg of oxalic acid in 2-propanol (2.5 mL). The resulting mixture was heated to 75 to 80°C, stirred for 30 minutes, allowed to cool to ambient temperature (about 20°C), stirred for 18 hours, and then filtered. The resulting cake was washed with 5 mL of 2-propanol and with 5 mL of methyl tert-butyl ether respectively

and then dried, affording 0.08 g (0.27 mmol: overall yield from nitrile (h) : 25 %) of hydrogenoxalate of tert-butyl (j) as a white solid.

Example 31 (Step E)

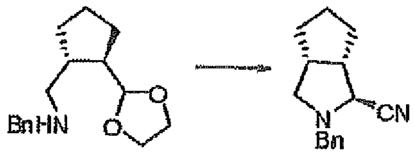

(e2) (h2)

To a solution prepared by dissolving 10.8 g (41.4 mmol) of protected acetal (e2) in 2-propanol (69 mL) was added a mixed liquid of water (6.9 mL) and 6.9 mL (82.8 mmol) of 35% hydrochloric acid. The mixture was stirred for 48 hours and then it was confirmed with HPLC that protected acetal (e2) was converted. After adding 31.5 g (207 mmol) of DBU, the resulting mixture was stirred at room temperature for 20 hours. The stirred mixture was cooled to 0°C and then a solution prepared by dissolving 2.4 g (49.7 mmol) of sodium cyanide in water (13.8 mL) was dropped over 20 minutes. After stirring at that temperature for 1 hour, a 10% aqueous potassium carbonate solution (69 mL) was added. This mixture was subjected to extraction with 69 mL of methyl tert-butyl ether three times. The ether layers were combined and dried over magnesium sulfate. The solvent was evaporated under reduced pressure, followed by purification of respective isomers of nitrile (h2) (nitrile compound (7)) with silica gel column chromatography (ethyl acetate: heptane = 40 : 1), affording 4.82 g of the exo form and 2.18 g of the end form (combined yield: 74%). $^1$H-NMR($CDCl_3$) δ: 7.35-7.22 (5H, m), 3.84 (1H, d, J=13.2 Hz), 3.61 (1H, d, J=13.2 Hz), 3.43-3.41 (1H, m), 2.81-2.75 (1H, m), 2.74-2.70 (1H, m), 2.67-2.60 (2H, m) 1.92-1.81 (2H, m), 1.78-1.69 (1H, m), 1.48-1.36(3H, m)
$^{13}$C-NMR($CDCl_3$) δ: 137.8, 128.5, 128.4, 127.3, 117.6, 59.6, 58.5, 55.8, 48.3, 41.2, 34.3, 33.5, 27.2

Example 32 (Step F)

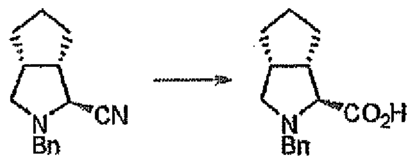

(h2) (k2)

To 3.8 g (16.8 mmol) of nitrile (h2) was added 35% hydrochloric acid (114 mL) and the resulting mixture was stirred at 75°C for 15 hours, followed by cooling to room temperature. After adding toluene (38 mL), the resulting viscous solution was stirred until the solution separated into layers. After separating the aqueous layer, this aqueous layer was subjected to extraction with toluene (38 mL) twice. The toluene layers were combined and then the solvent was evaporated under reduced pressure. The residue was dissolved in water (38 mL) and then a 1% aqueous sodium hydroxide solution was added to adjust pH to about 7. After conducting extraction with 1-butanol (38 mL) three times, the organic layers were combined and then the solvent was evaporated under reduced pressure, affording 4.0 g (yield: 98%) of protected amino acid (k2).
$^1$H-NMR($CDCl_3$) δ: 8.34 (1H, s), 7.46-7.35 (5H, m), 4.65 (1H, d, J=13.2 Hz), 4.05 (1H, d, J=13.2 Hz), 3.73 (1H, dd, J=10.7, 8.3 Hz), 3.22 (1H, d, J=9.3Hz), 3.05-2.97 (1H, m), 2.84-2.78 (1H, m), 2.26 (1H, t, J=10.2 Hz), 1.98-1.88 (1H, m), 1.76-1.63(2H, m), 1.62-1.52 (1H, m), 1.47-1.34 (2H, m)
$^{13}$C-NMR($CDCl_3$) δ: 170.6, 130.6, 129.9, 129.4, 129.1, 73.2, 57.1, 56.4, 47.5, 39.7, 31.9, 30.9, 24.0

Example 33 (Step G)

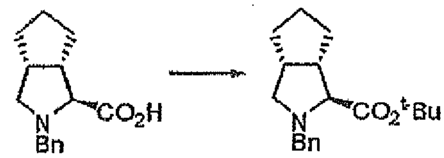

(k2) (l2)

A solution prepared by dissolving 0.5 g (2.0 mmol) of protected amino acid (k2) and 0.7 mg (7.7 mmol) of MsOH in chloroform (25 mL) was stirred at room temperature for 18 hours with isobutylene bubbled. After adding a 20% aqueous sodium hydroxide solution (25 mL), the resulting viscous solution was stirred for 1 hour. The chloroform layer was removed by separation, and the aqueous layer was subjected to extraction with chloroform (25 mL) twice. The chloroform layers were combined and dried over magnesium sulfate. The solvent was evaporated under reduced pressure, and the resulting residue was purified with silica gel column chromatography (ethyl acetate : heptane = 10 : 1), affording 0.6 g (yield: 99%) of the objective protected ester (12) as a colorless oil.
$^1$H-NMR($CDCl_3$) δ: 7.34-7.22(m, 5H), 3.96(d, J=12.7 Hz, 1H), 3.32-3.29(m, 1H), 3.19-3.15(m, 1H), 2.70-2.60 (m, 3H), 1.91-1.81 (m, 1H), 1.75-1.52 (m, 6H), 1.49 (s, 9H) $^{13}$C-NMR($CDCl_3$) δ: 172.7, 138.3, 129.1, 128.1, 127.0, 80.6, 73.2, 59.9, 58.0, 48.6, 41.0, 31.9, 31.8, 28.2, 24.9

Example 34 (Step G)

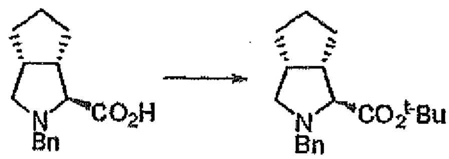

(k2) (l2)

After raising the temperature of a solution prepared by dissolving 2.00h (8.15 mmol) of protected amino acid (k2), 49.8 mg (0.41 mmol) of N,N-dimethylaminopyridine, and 1.21 g (16.31 mmol) of tert-BuOH in tert-butyl methyl ether (15 mL) to 35°C, di-tert-butyl dicarbonate (12.23 mmol) was dropped and stirred for 24 hours. (Reaction Yield: 97%)*) The resulting reaction mixture solution was washed with water (4 g) once, and with a 15% aqueous sodium chloride solution (4 g) once. The solvent of the resulting organic layer was evaporated under reduced pressure, and the resulting residue was purified with silica gel column chromatography (ethyl acetate : heptane = 20 : 1), affording 2.16 g (yield: 88%) of the objective protected ester (12) as a pale yellow oil.
*) Quantitative analysis was conducted under the following conditions by using HPLC.

[HPLC conditions]

Column: CAPCELL PACK C8 DD, 4.6 mmϕ × 150 mm, 5 μm (produced by Shiseido)
Flow rate: 1.00 ml/min
Detection wavelength: UV 210 nm
Mobile phase: Liquid A (0.1% TFA) /Liquid B (acetonitrile)
Gradient conditions:

| Time (minute) | 0 | 25 | 45 |
|---|---|---|---|
| Liquid A | 90 | 20 | 20 |
| Liquid B | 10 | 80 | 80 |

Column temperature: 30°C

Example 35 (Step G)

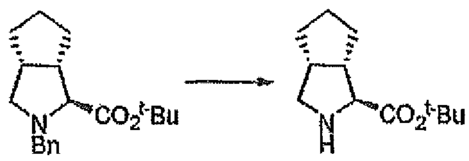

(I2) (j)

A mixture of 0.5 g (1.6 mmol) of protected ester (12), 0.27 g of 20% $Pd(OH)_2$/carbon, and methanol (25 mL) was stirred at 30°C under a hydrogen overpressure (500 kPa) for 2 hours. The reaction mixture was filtered under a nitrogen atmosphere through a filter precoated with celite and the solvent was evaporated from the filtrate under reduced pressure, affording 0.33 g (yield: 98%) of ester (j).
$^1$H-NMR($CDCl_3$) $\delta$: 3.33-3.28 (1H, m), 3.15 (1H, d, J=5.9 Hz), 2.62-2.50 (2H, m), 2.48-2.44 (1H, m), 2.13 (1H, s), 1.80-1.68 (1H, m), 1.66-1.55 (5H, m), 1.47(9H, s)
$^{13}$C-NMR($CDCl_3$) $\delta$: 173.7, 80.6, 68.1, 53.9, 49.7, 44.2, 32.3, 32.1, 28.0, 25.3

Example 36 (Step G)

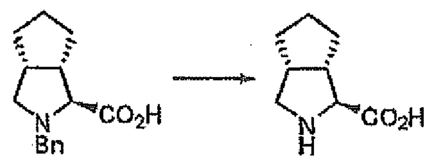

(k2) (I2)

A mixture of 0. 5 g (2.0 mmol) of protected amino acid (k2), 0.34 g of 20% $Pd(OH)_2$/carbon, and methanol (30 mL) was stirred at 30°C under a hydrogen overpressure (500 kPa) for 2 hours. The reaction mixture was filtered under a nitrogen atmosphere through a filter precoated with celite and the solvent was evaporated from the filtrate under reduced pressure, affording a green solid. This solid was recrystallized from a mixed solvent of methanol and methyl tert-butyl ether, affording 0.28 g (yield: 90%) of purified amino acid (i2).
$^1$H-NMR($CDCl_3$) $\delta$: 3.57 (d, J=6.84 Hz, 1H), 3.51-3.46 (m, 1H), 2.85-2.70 (m, 3H), 182-1.75(m, 1H), 1.70-1.59 (m, 4H), 1.42-1.30 (m, 1H)
$^{13}$C-NMR($D_2O$) $\delta$: 175.6, 68.5, 52.1, 48.8, 43.3, 33.4, 32.4, 26.0

Example 37 (Step G)

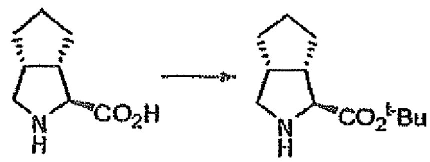

(i2) (j)

A solution prepared by dissolving 0.16 g (1.0 mmol) of amino acid (i2) and 0.36 mg (3.8 mmol) of methanesulfonic acid in chloroform (1.2 mL) was cooled to 0°C, and then 3.4 g of isobutylene was added and sealed. The temperature was returned to room temperature and the mixture was stirred at this temperature for 18 hours, followed by cooling to 0°C again. After adding a 50% aqueous sodium hydroxide solution (1.32 mL) carefully, 2.0 mL of water kept at 20°C was added, followed by stirring for 15 minutes. The chloroform layer was removed by separation, and the aqueous layer was subjected to extraction with chloroform (20 mL) three times. The chloroform layers were combined and dried over magnesium sulfate. The solvent was evaporated under vacuum, and the resulting residue was purified with silica gel column chromatography (ethyl acetate : methanol = 1 : 1), affording 0.18 g (yield: 85%) of the objective ester (j) as a green oil.

INDUSTRIAL APPLICABILITY

According to the present invention, there is provided a novel process for producing an ester compound represented by Formula (1) that is useful as an intermediate to produce medicines, agrochemicals, and the like.

## Claims

1. A process for producing an ester compound represented by Formula (1) or a salt thereof

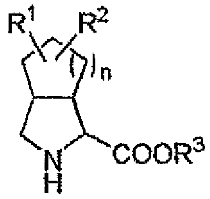

(1)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^3$ represents a protective group of a carboxyl group, and n represents 1 or 2, the process comprising Steps A to G defined below:

[Step A] a step of reacting an aldehyde compound represented by Formula (2)

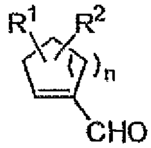

(2)

wherein $R^1$, $R^2$, and n are each as defined above,
with nitromethane in the presence of a pyrrolidine compound to produce a (nitroaldehyde compound represented by Formula (3);
[Step B] a step of reacting the nitroaldehyde compound represented by Formula (3)

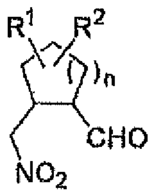

(3)

wherein $R^1$, $R^2$, and n are each as defined above,
with an alcohol to produce a nitroacetal compound represented by Formula (4):
[Step C] a step of reducing the nitroacetal compound represented by Formula (4)

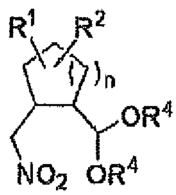

(4)

wherein $R^1$, $R^2$, and n are each as defined above, and $R^4$s each represent an alkyl group or the two $R^4$s are combined together to represent an alkylene group,
to produce an aminoacetal compound represented by Formula (5) or a salt thereof;
[Step D] a step of protecting an amino group in the aminoacetal compound represented by Formula (5)

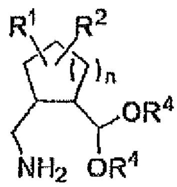 (5)

wherein $R^1$, $R^2$, $R^4$, and n are each as defined above,
or a salt thereof to produce a protected aminoacetal compound represented by Formula (6) or a salt thereof;
[Step E] a step of treating the protected aminoacetal compound represented by Formula (6)

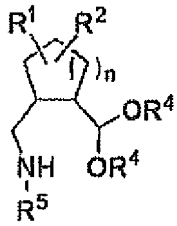 (6)

wherein $R^1$, $R^2$, $R^4$, and n are each as defined above, and $R^5$ represents a protecting group of an amino group, or a salt thereof with an acid and subsequently with a base and then reacting the resultant product with a cyanating agent to produce a nitrile compound represented by Formula (7) or a salt thereof;
[Step F] a step of hydrolyzing the nitrile compound represented by Formula (7)

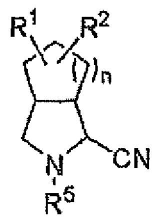 (7)

wherein $R^1$, $R^2$, $R^5$, and n are each as defined above,
or a salt thereof to produce a protected amino acid compound represented by Formula (8) or a salt thereof; and
[Step G] a step of substituting a group represented by $R^5$ contained in the protected amino acid compound represented by Formula (8)

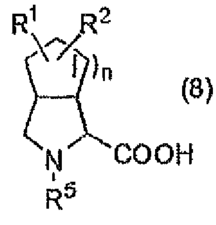 (8)

wherein $R^1$, $R^2$, $R^5$, and n are each as defined above,
or a salt thereof by a hydrogen atom and protecting a carboxyl group therein.

**2.** The production process according to claim 1, wherein Step A is a step of reacting the aldehyde compound represented

by Formula (2) with nitromethane further in the presence of water.

3. The production process according to claim 2, wherein Step A is a step of reacting the aldehyde compound represented by Formula (2) with nitromethane further in the presence of a carboxylic acid.

4. The production process according to any of claims 1 to 3, wherein the pyrrolidine compound in Step A is a compound represented by Formula (9)

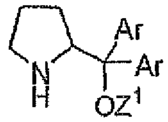
(9)

wherein Ar represents an optionally substituted aryl group, and $Z^1$ represents an alkyl group or a trialkylsilyl group (each of the alkyl groups in the trialkylsilyl group may be the same or different).

5. The production process according to claim 4, wherein Ar is phenyl and $Z^1$ is a trimethylsilyl group.

6. The production process according to any of claims 1 to 3, wherein the aldehyde compound represented by Formula (2) is 1-formylcyclopentene.

7. A process for producing a protected aminoalcohol compound represented by Formula (6bX)

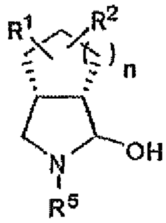
(6bX)

wherein $R^1$, $R^2$, $R^5$, and n are each as defined below, the process comprising a step of treating a protected aminoacetal compound represented by Formula (6X)

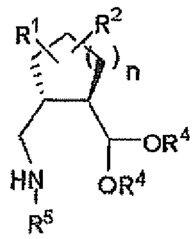
(6X)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, $R^5$ represents a protecting group of an amino group, and n represents 1 or 2,
or a salt thereof with an acid to form a protected aminoaldehyde compound represented by Formula (6aX)

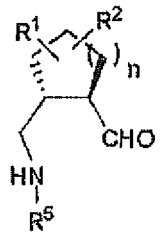

(6aX)

wherein $R^1$, $R^2$, $R^5$, and n are each as defined above, and a step of treating the protected aminoaldehyde compound represented by Formula (6aX) with a base.

**8.** The production process according to claim 7, wherein the base is 1,8-diazabicyclo[5.4.0]undec-7-ene or a metal alkoxide.

**9.** A nitroacetal compound represented by Formula (4)

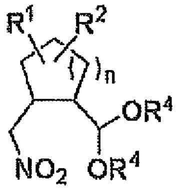

(4).

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, and n represents 1 or 2.

**10.** An aminoacetal compound represented by Formula (5)

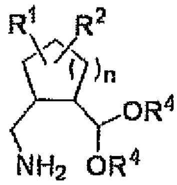

(5)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, and n represents 1 or 2,
or a salt thereof.

**11.** A protected aminoacetal compound represented by Formula (6)

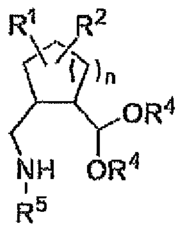

(6)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,
or a salt thereof.

**12.** A nitrile compound represented by Formula (7)

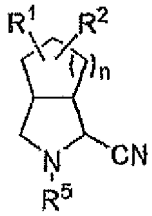

(7)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^4$s each represent an alkyl group or the two $R^4$s are combined together to represent an alkylene group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,
or a salt thereof.

**13.** A protected amino acid compound represented by Formula (8)

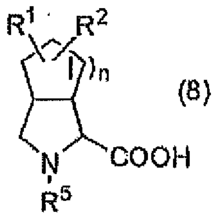

(8)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,

or a salt thereof.

**14.** A protected aminoaldehyde compound represented by Formula (6a)

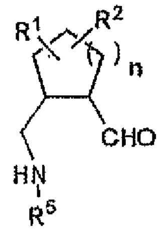

(6a)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,
or a salt thereof.

**15.** A protected aminoalcohol compound represented by Formula (6b)

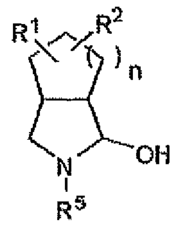

(6b)

wherein $R^1$ and $R^2$ each independently represent a hydrogen atom, an optionally substituted alkyl group, an optionally substituted alkoxy group, an optionally substituted alkenyl group, an optionally substituted alkenyloxy group, an optionally substituted cycloalkyl group, an optionally substituted cycloalkoxy group, an optionally substituted aryl group, an optionally substituted heteroaryl group, a halogen atom, an optionally substituted alkoxycarbonyl group, or an optionally substituted amino group, $R^5$ represents a protecting group of an amino group, the protecting group being an optionally substituted alkyloxycarbonyl group, an optionally substituted aryloxycarbonyl group, or an optionally substituted aralkyl group, and n represents 1 or 2,
or a salt thereof.

**INTERNATIONAL SEARCH REPORT**

International application No.
PCT/JP2012/063243

A. CLASSIFICATION OF SUBJECT MATTER
*C07D209/52*(2006.01)i, *C07C205/29*(2006.01)i, *C07C217/44*(2006.01)i, *C07C271/16*(2006.01)i, *C07C271/18*(2006.01)i, *C07D317/16*(2006.01)i, *C07D317/28*(2006.01)i

According to International Patent Classification (IPC) or to both national classification and IPC

B. FIELDS SEARCHED

Minimum documentation searched (classification system followed by classification symbols)
C07D209/52, C07C205/29, C07C217/44, C07C271/16, C07C271/18, C07D317/16, C07D317/28

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2004/092134 A1 (WARNER-LAMBERT CO. L.L.C.), 28 October 2004 (28.10.2004), compound example B4, steps (3), (4); compound example F1, step (7); compound example N1, step (2) & US 2005/137215 A1 & EP 1615886 A1 & MX 2005010165 A1 & BR 200409428 A | 12,15 |
| X | YASUDA,M. et al., Synthesis of conformationally defined glutamic acid analogs from readily available Diels-Alder Adducts, Chemical & Pharmaceutical Bulletin, 1995, Vol.43, No.8, p.1318-1324, compounds 17b, 18b | 12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 August, 2012 (01.08.12) | 14 August, 2012 (14.08.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/063243

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JAO,E. et al., [3+2] Cycloaddition of trimethylenemethane (TMM) to α,β-unsaturated γ-lactam. Preparation of 5,5-fused proline surrogates, Tetrahedron Letters, 2003, Vol.44, No.27, p.5033-5035, compounds 4a, c to e | 13<br>10,11 |
| X<br>Y | JP 2008-538563 A (Merck & Co., Inc.),<br>30 October 2008 (30.10.2008),<br>example 5, step A<br>& WO 2006/115918 A2 & EP 1874299 A2<br>& AU 2006240146 A1 & IN 200704505 P4<br>& CN 101163472 A & CA 2604531 A1<br>& US 2009/069376 A1 | 13<br>10,11 |
| X | PUNNIYAMURTHY,T. et al., Asymmetric desymmetrization of meso-pyrrolidine derivatives by enantiotopic selective CH hydroxylation using (salen)manganese(III) complexes, Tetrahedron, 1999, Vol.55, No.31, p.9439-9454, compound 19b | 15 |
| X<br>A | JP 2008-546838 A (Merck & Co., Inc.),<br>25 December 2008 (25.12.2008),<br>compounds 35 to 37, 78 to 80 (claim 8)<br>& WO 2007/02457 A2 & EP 1898905 A2<br>& AU 2006261930 A1 & CA 2611550 A1<br>& US 2009/054462 A1 & US 7683068 B2<br>& AU 2006261930 B2 | 15<br>1-9,14 |
| Y<br>A | US 4254057 A (SHELL OIl CO.),<br>03 March 1981 (03.03.1981),<br>Preferred Embodiments; example 1<br>& EP 7652 A & DK 7902666 A<br>& JP 55-07278 A & BR 7903987 A<br>& ZA 7903152 A & US 4255334 A<br>& US 4279821 A & EP 7652 B<br>& CA 1117127 A & DE 2962088 G | 10,11<br>1-9,14 |
| A | JP 2007-520434 A (Schering Corp.),<br>26 July 2007 (26.07.2007),<br>entire text<br>& WO 2004/113295 A1 & US 2005/059648 A1<br>& EP 1633711 A1 & MX 2005013752 A1<br>& CN 1805931 A & US 7309717 B2<br>& US 2008/114179 A1 & US 7569705 B2<br>& MX 262939 B & US 2009/247784 A1<br>& US 7728165 B2 & CN 1805931 B<br>& CN 101823965 A & EP 1633711 B1<br>& DE 602004030239 E & MX 284484 B<br>& HK 1081199 A1 & ZA 200509105 A<br>& ZA 201006378 A & JP 4825129 B2 | 1-9,14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2007022459 A
- WO 2010126881 A

### Non-patent literature cited in the description

- **T. W. GREENE ; P. G. M. WUTS.** Protective Groups in Organic Synthesis. Wiley-Interscience, 2006